# EUROPEAN PATENT APPLICATION

(11) **EP 4 804 206 A1**
(43) Date of publication of application: **09.09.2026**
(21) Application number: 24886353.2
(22) Date of filing: 01.11.2024
(51) Int. Cl.: G16H 50/20, G16B 30/00, G16B 25/00, G01N 33/68, G16B 50/00, G16H 15/00, G16H 10/60

(54) **NAIL-BASED NGS DATA ANALYSIS METHOD AND SYSTEM FOR DISEASE PREDICTION**

(30) Priority: 03.11.2023 KR 20230151141
(71) Applicant: Metealth Co., Ltd., Yongin-si, Gyeonggi-do 16957 (KR)
(72) Inventor: KWON, Chunga, Seongnam-si Gyeonggi-do 13163 (KR); LEE, Eojin, Yongin-si Gyeonggi-do 16957 (KR)
(74) Representative: Fuchs Patentanwälte Partnerschaft mbB
(86) International application number: PCT/KR2024/017050
(87) International publication number: WO 2025/095682

(57) **Abstract**

The present invention relates to a next generation sequencing (NGS) method for disease prediction through analysis of nail-derived germline mutations and somatic mutations, and a system therefor. Specifically, the present invention relates to a nail-based NGS analysis method for predicting or diagnosing a disease, comprising the steps of: isolating gDNA from nails; and analyzing the isolated gDNA using NGS.

## Description

### [Technical Field]

The present invention relates to a next generation sequencing (NGS) method and system for disease prediction through the analysis of nail-derived germline mutations and somatic mutations. Specifically, the present invention relates to a nail-based NGS (Next Generation Sequencing) analysis method for the prediction or diagnosis of diseases, comprising the steps of isolating gDNA from a nail and analyzing the isolated gDNA using NGS.

### [Background Art]

Precision medicine involves identifying individual variations in genetic and environmental factors, medical history, and lifestyle to provide optimized treatment by administering the right drug at the right dose and at the right time for each patient. In particular, as prevention is considered superior to reactive treatment, personal genome analysis serves as a key element in realizing future medical paradigms such as precision and preventive medicine, enabling the preemptive identification and management of specific diseases or health risks.

While large-scale studies such as whole genome sequencing (Whole Genome-seq), transcriptome sequencing (Transcriptome-seq), and epigenome sequencing (Epigenome-seq) are being conducted by various research groups to identify the genetic correlations between complex traits and diseases, several limitations such as nonrandom sampling, disease complexity, the lack of bio big data, and the need for advancements in bioinformatics analysis methods still persist. However, the necessity and utility of personal genome analysis are projected to continue growing, driven by the research achievements in clinical genomics.

Since the COVID-19 crisis, it has been revealed that individuals with underlying conditions such as hypertension and diabetes are more likely to become critically ill upon infection, which has led to increased awareness of routine health management. In particular, as obesity rates rise, chronic diseases accompanied by hypertension are further increasing, and in the case of Korea, most diabetic patients are exhibiting dyslipidemia.

Hyperlipidemia refers to a condition in which blood cholesterol and blood triglyceride levels are abnormally high, and dyslipidemia is a broad concept that includes such abnormalities in blood lipid levels, with one in four adults in Korea currently suffering from hypercholesterolemia and two in five suffering from dyslipidemia. It is reported that hypercholesterolemia is on an increasing trend, with 23% of men and 25% of women having hypercholesterolemia, and while awareness is improving accordingly, three out of ten hypercholesterolemia patients are still unaware of their condition, and although the treatment rate has improved significantly, only about half of hypercholesterolemia patients are actually taking lipid-lowering agents. Although a majority of diabetic patients in Korea have hyperlipidemia, the awareness and treatment rates of these patients are only in the 20-30% range, indicating an urgent need for improvement. It was found that 83.3% of adult diabetic patients had hyperlipidemia, with 88.3% for women and 78.1% for men, showing that women were higher than men, and in particular, the prevalence among the young population aged 19-39 was 88.5%, which was higher than other age groups. This figure is much higher than the hyperlipidemia prevalence (15-20%) of the general population in their 20s and 30s reported in existing studies, suggesting that for diabetic patients, management of hyperlipidemia is necessary from an earlier age (Seung Jae Kim, Oh Deog Kwon, Kyung-Soo Kim, Prevalence, awareness, treatment, and control of dyslipidemia among diabetes mellitus patients and predictors of optimal dyslipidemia control: results from the Korea National Health and Nutrition Examination Survey. Lipids Health Dis. 2021 Mar 26;20(1):29.).

Additionally, chronic diseases commonly accompanied by cancer survivors include hypertension, hyperlipidemia, diabetes, osteoporosis, and anemia. Causative factors include genetic factors, lifestyle risk factors, the cancer itself, cancer treatment, and pre-existing chronic diseases, and as a result of analyzing the causes of death of approximately 240,000 long-term cancer survivors who were diagnosed with cancer between 1993 and 2000 and survived for more than five years in Korea, 24% of cancer survivors died from causes other than cancer, most of which were diabetes and cardiovascular diseases (Dong Wook Shin, Young Ho Yun et al., Non-cancer mortality among long-term survivors of adult cancer in Korea: national cancer registry study. Cancer Causes Control 2010; 21: 919-29.). Besides, chronic diseases such as hypertension, hyperlipidemia, osteoporosis, and anemia, which commonly occur at a higher frequency in cancer survivors, need to be checked more frequently than the screening intervals conducted for the general population.

To date, there are no guidelines or instructions on health checkup items for cancer survivors, and general health management guidelines are only being presented for commonly occurring solid cancer types. Cancer survivors have a higher risk of chronic diseases such as obesity, hypertension, diabetes, hyperlipidemia, osteoporosis, and anemia, and secondary cancer than the general population, which varies somewhat by cancer type, and they are a health-vulnerable group for whom smoking cessation, abstinence from alcohol, proper exercise, and nutritional management are very important, necessitating post-management and monitoring for prevention to predict the occurrence of secondary diseases.

Therefore, in order to fundamentally prevent and treat diseases, an approach to identifying genetic status and mutations tailored to individual characteristics is necessary. Sanger-based sequencing, a conventional sequencing method, can generally read DNA fragments within 500-800 base pairs. Thus, it is limited in analyzing long DNA sequences, and its application to research fields requiring large-scale DNA sequence information, such as personal genome analysis, involves disadvantages such as inefficiency in terms of time, labor, and cost. For these reasons, as a new analysis technique capable of obtaining large-scale DNA sequence information with high efficiency and low cost is required, the popularization of next generation sequencing (NGS) and technologies using the same are developing rapidly.

The detection of variations within a genome highly depends on the performance of variant calling software. While various software is currently in use, significant differences exist in the variant calling capabilities of each software. To address this, various studies are being conducted to find an optimal standard pipeline, but the software that demonstrates the best performance may vary depending on the characteristics or quality of the input data.

In particular, Mutect2, which is representatively and widely used among variant detection software, is known to demonstrate excellent performance in low-frequency variants, but cases of false negatives have been reported in clinical settings. Various additional options are provided to overcome this problem, but in some cases, there are limitations in failing to detect clinically important variants. Since software such as Mutect2 uses probabilistic models to calculate the probability of the existence of a variant, there is a possibility that errors may occur during this probability calculation process, which can degrade the reliability of variant detection. Therefore, there is an urgent need for a new approach to increase the accuracy of variant detection and resolve the false negative issue.

As multinational corporations develop NGS equipment and improve reagents, and as bioinformatics techniques advance, the cost of genotyping is gradually becoming more affordable. NGS analysis has various advantages that can replace the conventional Sanger-based methods, and thus it is being utilized in many research, health checkup, and diagnostic service fields.

As a result, tissue and blood samples have traditionally been used as the primary sources of DNA for genetic analysis, but tissue samples have limitations in collection through surgery or procedures, and in the case of blood collection, there are difficulties in collecting specimens due to factors such as arterial thrombosis, skin infection, anemia, or hemophilia patients where blood collection is impossible, persons who have not passed 4 weeks after receiving drugs such as isotretinoin, women during pregnancy or for a certain period after childbirth, and patients with infectious diseases or leukemia.

Additionally, such tissue or blood specimens, upon moving to a place for analysis or testing, specimen transport instability according to temperature changes and high-cost transportation fee burden for maintaining refrigeration or freezing, and freezing or ultra-low temperature equipment and facilities in terms of long-term storage are being required as essential construction items, and due to people such as children or adults or patients who, depending on the case, are suffering from needle phobia or trypanophobia which is a rejection of injection needles, a system that can resolve the above disadvantages from alternative clinical specimens and can screen and diagnose more periodically and smoothly is being required.

In order to solve such problems, the present inventors have completed the present invention by developing an analysis method and system with high accuracy and a reduced possibility of false negatives through nail specimens that can replace blood or tissues.

### [Prior Art Documents]

### [Non-Patent Documents]

Seung Jae Kim, Oh Deog Kwon, Kyung-Soo Kim, Prevalence, awareness, treatment, and control of dyslipidemia among diabetes mellitus patients and predictors of optimal dyslipidemia control: results from the Korea National Health and Nutrition Examination Survey. Lipids Health Dis. 2021 Mar 26;20(1):29.

Dong Wook Shin, Young Ho Yun et al., non-cancer mortality among long-term survivors of adult cancer in Korea: national cancer registry study. Cancer Causes Control 2010; 21: 919-29.

Barbitoff, Y.A., Abasov, R., Tvorogova, V.E. et al. Systematic benchmark of state-of-the-art variant calling pipelines identifies major factors affecting accuracy of coding sequence variant discovery. BMC Genomics 23, 155 (2022).

### [Summary of Invention]

### [Technical Problem]

The objective of the present invention is to provide an NGS-based genetic data analysis method and system capable of predicting the presence or absence of a disease according to individual characteristics, which can be easily implemented in health checkup centers, medical institutions, and future DTC (Direct to Consumer) testing services by utilizing nail (fingernail or toenail) specimens.

### [Technical Solution]

The present invention provides a nail-based NGS analysis method for predicting or diagnosing a disease and a system for predicting or diagnosing a disease using the same.

Specifically, the present invention provides a nail-based NGS analysis method capable of diagnosing or predicting chronic diseases such as obesity, hyperlipidemia, and diabetes, the occurrence, recurrence, and metastasis of cancer, and a system for predicting or diagnosing a disease using the same.

The present invention provides the nail-based NGS analysis method for predicting or diagnosing the disease, comprising the steps of: (a) isolating gDNA from a nail; and (b) analyzing the gDNA isolated in step (a) by NGS (Next-Generation Sequencing).

The step (a) of isolating gDNA from the nail is a step of preparing a sample for PCR or NGS analysis by applying a gDNA extraction based on a forensic method used in forensic science, rather than applying a conventional general extraction method using phenol/chloroform, in order to reduce DNA damage in the nail specimen.

The nail includes a fingernail, a toenail, or both a fingernail and a toenail. Furthermore, the nail is not limited to those of humans and includes all those of animals (or subjects) having fingernails or toenails, such as dogs and cats.

The growth rate of nails may vary by individual, but on average, they typically grow from 1.8 mm to a maximum of 4.5 mm per month, and a characteristic is that increased stimulation leads to higher blood flow to the nails, activating cell division and resulting in faster growth. Among other relatively non-invasive specimens, hair typically grows at an average rate of 0.3 mm per day, about 1 cm per month, and approximately 12 cm per year; however, this growth rate is slower compared to nails, and in cases of children, individuals without hair, or trace DNA extraction resulting from small hair samples, variables may occur in NGS analysis results. Similarly, in the case of saliva, results can be adversely affected by factors such as the use of mouthwash, foreign substances ingested at the time of collection, excessive water intake, or oral microorganisms; furthermore, it requires a disposable saliva collection kit and a tube containing a separate preservative, and limitations may arise in specific genomic research or diagnosis due to the detection of only trace amounts of DNA. Therefore, the collection of nail specimens which allow for transport and storage at room temperature and have no restrictions regarding age, gender, or specific diseases is the most suitable for analysis.

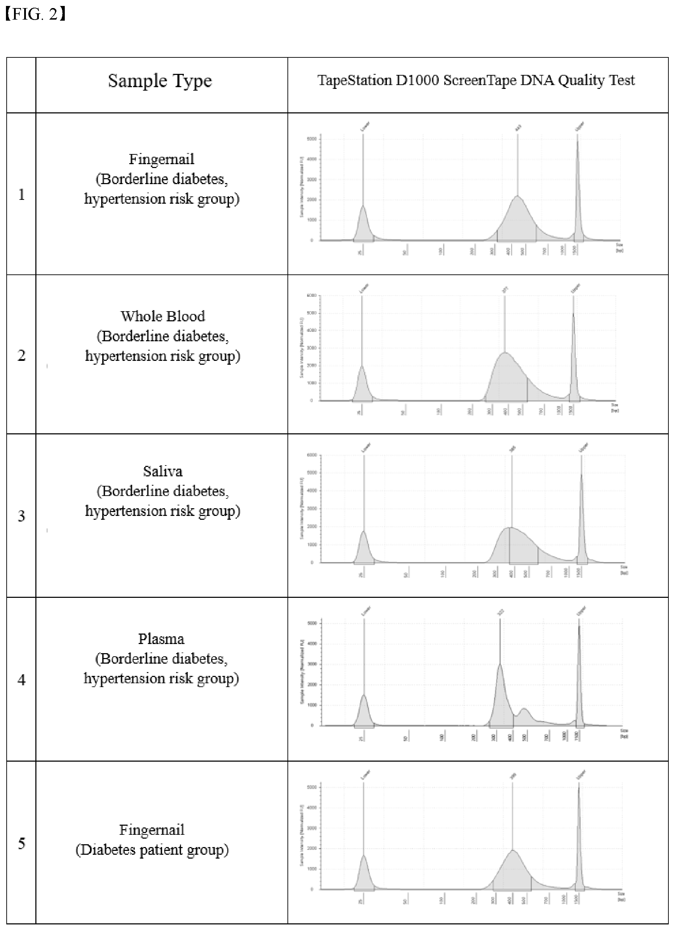

Through the experimental results shown in FIG. 2, the present inventors confirmed that stable gDNA extraction is possible from nails, just as with other specimens, and the samples were directly collected by the requester at room temperature, stored in paper containers, and then applied to the tests. It was confirmed that nail specimens can be suitable samples for genetic testing as they can be utilized from screening to diagnosis at screening centers and medical institutions by increasing requester convenience in an eco-friendly manner.

As for the collection method, nail samples from individuals or patients can be collected and provided by genetic testing agencies, health checkup centers, clinics, or primary or secondary hospitals in each region. By testing the gDNA (genomic DNA) contained in nail samples, the occurrence of chronic diseases such as aging, obesity, hyperlipidemia, and diabetes, as well as related complications, can be predicted, and the possibility of cancer occurrence, metastasis, and recurrence can be reviewed.

Additionally, the NGS-based data analysis method of the present invention may include a step of obtaining data by receiving existing clinical information from screening agencies or primary to secondary hospitals to enable comparison with existing blood samples of the analysis subject.

For gDNA extraction from nail samples, single nucleotide polymorphism (SNP) genotyping can be performed from nail specimens using phenol/chloroform extraction. As a common method for extracting DNA from various samples including nails, the phenol/chloroform extraction method degrades the cell membranes in the nail sample to release DNA and then extracts the DNA from the solution using salt precipitation.

While phenol/chloroform extraction is a relatively simple and efficient method for extracting DNA from nails, it has the disadvantages of being time-consuming and potentially damaging the DNA. Furthermore, the phenol/chloroform extraction method poses a problem where DNA may also be extracted from other sources, such as bacteria and fungi. This can lead to the contamination of the DNA sample. While the phenol/chloroform extraction method may be used when nail-derived DNA is required only for SNP genotyping, a more efficient and less damaging method is necessary if the DNA is required for other applications such as NGS sequencing or PCR (polymerase chain reaction).

SNP genotyping is a technique used to identify SNPs in DNA samples. SNPs result from variations of a single nucleotide (A, T, C, or G) in a DNA sequence, and they can be used to identify individuals, track genetic variation in populations, and diagnose genetic diseases.

In NGS, if impurities such as salts, phenol, ethanol, or iron are mixed in the sample, or if substances such as heparin, EDTA, NaCl, or KCl are introduced during the DNA extraction stage, the results may be interfered with, leading to inaccurate conclusions; thus, it is necessary to identify the causes of errors before testing.

To reduce such DNA damage, the present invention extracted gDNA by applying a forensic method instead of the extraction method using phenol/chloroform. Accordingly, time-consuming washing steps, such as precipitation using isopropanol or ethanol, are omitted, thereby shortening the extraction time and minimizing the damage rate.

In the NGS analysis step (b), NGS technology refers to a technique that fragments the DNA or RNA of an organism and reads the sequences using a machine. NGS technology involves extracting DNA, fragmenting the DNA into short sequence fragments, and performing sequencing to analyze the bases included in each sequence fragment. In one embodiment of the present invention, an Illumina sequencing method may be used as the NGS method, but it is not limited thereto.

The nail-based NGS analysis method of the present invention may further include the steps of:
(c) mapping genes from the gDNA isolated from the nail; and/or
(d) calculating the expression levels of genes associated with the onset of a disease.

The mapping step (c) refers to identifying the genomic location of each sequence fragment after sequencing the gDNA isolated from the nail by aligning each fragment based on a reference genome. In this context, a reference genome is a virtual nucleotide sequence of an individual representing a biological species, serving as a genomic map that guides the mapping process. After identifying the locations of all sequence fragments, various analyses can be performed, such as analyzing DNA mutations or measuring the amount of DNA transcribed into RNA.

The calculation step (d) refers to calculating the expression of genes associated with the onset of a disease by obtaining probabilities that the analysis subject data corresponds to each of the genotypes related to the analysis subject gene, based on the mapping results of step (c). Here, "analysis subject data" refers to genomic sequence data obtained through an individual's nail-based NGS (Next-Generation Sequencing) analysis. This data includes information necessary for identifying individual genetic characteristics, including genetic variations related to specific diseases or health conditions. Additionally, "analysis subject genes" are genes known to be associated with the onset of specific diseases; for example, they can be used to evaluate the impact on disease susceptibility by analyzing genotypes at specific SNP (Single Nucleotide Polymorphism) positions.

The calculation step (d) includes identifying positions and sequences with variations or detecting and identifying somatic or germline mutations based on the mapping results of step (c). The predicted results can be comparatively analyzed with results from Whole Genome Sequencing (WGS), which analyzes the entire genome; Whole Exome Sequencing (WES), which examines the protein-coding regions of all genes; or targeted sequencing, which analyzes only specific genetic regions.

In the present invention, genomic information extracted from nails is read using the Whole Exome Sequencing technique, where it is most crucial to detect a small number of reads (DNA fragments whose base sequences are read by sequencing equipment) without omission even at a low depth. Accordingly, the present inventors developed and applied a variant caller algorithm (Bullseye) that identifies low-frequency variations by reading information from all reads.

The calculation step (d) according to the present invention includes aligning and mapping the sequenced reads to a reference genome and then identifying positions and sequences with variations based on the information of the mapped reads using a variant caller algorithm. Since the variant caller algorithm (Bullseye) of the present invention can be used to detect and identify differences between the reference genome and the reads, specifically somatic or germline mutations, Bullseye utilizes a method with improved accuracy through comparative validation with the existing Mutect2, reducing errors of false positives or false negatives.

Additionally, the nail-based NGS analysis method of the present invention may further include a step of (e) constructing a genomic database using the calculation results of step (d).

The step (e) of constructing a genomic database may involve building an individual's genomic information catalog based on result reports for disease prevention and prediction by identifying individual germline and somatic mutations.

Furthermore, the present invention provides a method of providing information for the prevention, diagnosis, or prognostic diagnosis of diseases based on an individual's germline and somatic mutations.

The genomic information catalog of step (e) may include SNP-based health information, which is a characteristic individual genotype; information related to exercise, nutrition, pharmacogenomics, immunity, skin, mental health, digestion, metabolic hormones, and metabolism; and disease-related content including the occurrence, recurrence, and metastasis of chronic diseases and cancer.

Specifically, the genomic information catalog may include genomic databases such as SNV, INDEL, and CNV obtained through nail-derived gDNA-based WGS (whole-genome sequencing), WES (whole-exome sequencing), and targeted sequencing from samples of healthy individuals or patients, as well as basic clinical data databases of patients with chronic diseases or cancer.

The present invention relates to a system for predicting or diagnosing diseases using the aforementioned NGS analysis method, comprising the following steps:
(a1) loading a computer program executed by one or more processors;
(b1) providing a memory that stores data in which genotypes for the genomic analysis subject are determined;
(c1) generating an individual genetic analysis result report based on the stored data; and
(d1) a system construction unit for building a genomic database of the analysis subject and a report generation step for providing information from the system construction unit to the analysis subject.

In the step (a1), the one or more processors may perform: (i) an operation step of receiving a nail sample, general information, and/or clinical information of the analysis subject to acquire and store data; (ii) a gDNA sample analysis operation step of mapping the analysis subject data to each of the nucleotide sequences with different genotypes regarding the analysis subject genome by tracking disease-related genes such as the occurrence of chronic diseases and/or the occurrence, recurrence, or metastasis of cancer in the nail sample, preferably in gDNA isolated from the nail; and (iii) a clinical information analysis operation step of calculating the expression levels of genotypes in the analysis subject data based on the mapping results.

The general information in (i) refers to information provided based on individual consent, such as age, gender, family history, or lifestyle; the clinical information refers to information such as blood pressure, blood glucose, smoking, drinking, disease history, vaccine and immunization information, regular checkup items (blood pressure, blood glucose, cholesterol, cancer screening, etc.), types and doses of prescribed drugs, side effect history, or allergic reactions, which may be provided by primary to secondary hospitals.

The system for predicting or diagnosing diseases according to the present invention may further include a step of (e) constructing a big data database after the report generation step (d1). The report may include an individual's constitution, customized nutrition and diet, exercise, stress relief and psychological/mental health management, recommended activities, combinations of health functional foods, supplements, regular health checkup cycles and methods, or trackable health goals and progress.

The system for predicting or diagnosing diseases according to the present invention can also be applied as a veterinary genetic testing method for animals with nails, such as dogs and cats.

In the present invention, diseases may include, but are not limited to, aging, obesity, hypertension, diabetes, hyperlipidemia, cancer, sarcopenia, osteoporosis, lung disease, cardiovascular disease, brain disease, liver disease, dementia, or Alzheimer's disease.

### [Advantageous Effects of Invention]

By complementing the disadvantages of conventional tissue-based and blood-based liquid biopsies, the individual genome analysis derived from nails according to the present invention not only enables the identification and proactive response to disease risks and health abnormalities but also makes precision medicine for personalized treatment possible.

As the incidence of diseases including obesity, aging, hyperlipidemia, diabetes, or cancer is increasing due to the interaction between genetic markers and environmental factors, disease prevention has become a matter of critical importance. Diagnostic technology using specimens that can be easily collected, stored, and transported in daily life, such as nail (fingernail or toenail) specimens, enables active health management to prevent and improve diseases in humans and animals. This can ultimately realize an efficient QOL (Quality of Life) by extending a healthy lifespan.

### [Brief Description of Drawings]

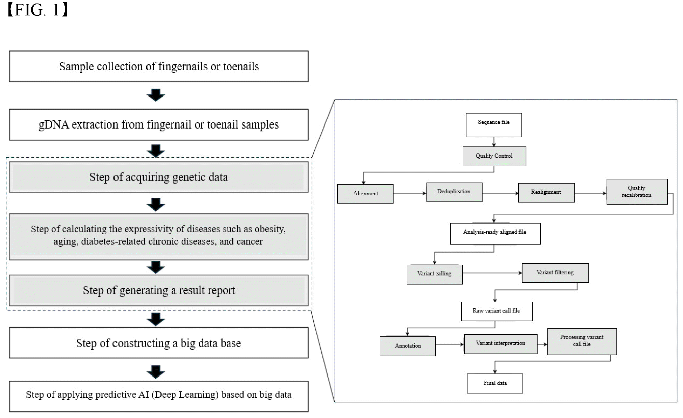

FIG. 1 illustrates an NGS-based data analysis system for disease prevention and prediction according to an individual's genetic characteristics, in accordance with an embodiment of the present invention.
FIG. 2 shows the results of a comparative analysis of nail-derived gDNA, (whole) blood-derived gDNA, plasma-derived cfDNA, and saliva-derived gDNA.
FIG. 3 shows the results of comparing the germline mutation detection rates of donors according to sample types.
FIG. 4 shows the results of comparing the somatic mutation detection rates of donors according to sample types.
FIG. 5 shows the results of mutation status related to disease diagnosis and treatment decisions in a diabetic patient group.
FIG. 6 shows the results of mutation status related to diagnosis and treatment decisions in a prediabetic patient group.
FIG. 7 shows the results of WES genetic mutation analysis related to sarcopenia when applying the developed in house caller (Bullseye) and the conventional variant caller (Mutect2) algorithms.
FIG. 8 shows the results of a comparative analysis of non-small cell lung cancer reference material (Seracare Tri-Level Tumor Mutation DNA Mix v2) when applying the developed in house caller (Bullseye) and the conventional variant caller (Mutect2) algorithms.
FIG. 9 shows the results of analyzing somatic variants in three types of specimens nail, blood, and surgical tissue targeting bladder cancer patients.

### [Best Mode for Carrying Out the Invention]

Hereinafter, embodiments and examples of the present invention will be described in detail with reference to the accompanying drawings so that those of ordinary skill in the art to which the present invention pertains can easily implement them. However, the present disclosure may be implemented in various different forms and is not limited to the embodiments and examples described herein.

Throughout the specification of the present disclosure, when a certain part is described as "comprising" a certain component, this means that it may further include other components rather than excluding other components, unless otherwise stated.

Hereinafter, the present invention will be described in more detail through examples; however, the following examples are for illustrative purposes only and are not intended to limit the scope of the present invention.

### [Example 1] Sample Preparation

### Isolation of gDNA from Nail Samples

In the present invention, in order to minimize damage during nail DNA extraction, an extraction method was applied based on Forensic DNA Kits (OMEGA BIO-TEK E.Z.N.A.^{®}, GeneAll Biotechnology Exgene^{™} Forensic SV mini, etc.) produced by domestic and international manufacturers, implementing a phenol/chloroform-free approach using approximately 10~20 mg of a single nail as a standard; and the volumes of the reaction solution and the reaction time for DNA elution were partially adjusted and applied to be suitable for nail specimens.

Sample QC was performed by verifying DNA concentration, purity, and fragment length; and the quality evaluation and analysis results for the genomic DNA (gDNA) samples were confirmed according to the TapeStation gDNA Screen Tape method, which is one of the microelectrophoresis analysis techniques.

Sample quality evaluation using the TapeStation is a method to quickly check the quality of gDNA samples, whereby it can be confirmed whether the samples are in a clean and intact state, and problems due to contamination or degradation can be identified. Additionally, since it is used to measure the concentration of gDNA, the amount of DNA included in the sample can be quantitatively calculated. Furthermore, it can visualize and analyze the size distribution of gDNA, making it possible to check the amount of DNA fragments in a specific size range or detect unusual phenomena in the size distribution; thus, it is suitable as a QC method for evaluating the quality of extracted DNA.

The samples used for comparative analysis were nail-derived gDNA from the same individual who was determined to be in the prediabetes and hypertension risk group through a general health checkup, and as control groups for comparison, (whole) blood-derived gDNA, plasma-derived cfDNA, and saliva-derived gDNA, which are currently mainly used in liquid biopsy. In addition, by comparing and analyzing with a nail-derived gDNA sample from a maternal donor who is a patient diagnosed with diabetes, DNA extraction results suitable for NGS analysis for the prevention and prediction of diabetes were derived through the observation of actual nail-derived germline mutations and somatic mutations, and the results are shown in Table 1 and FIG. 2 below.

**[Table 1]**

| | **Library name** | **Library Type** | **Concentration (ng/ul)** | **Concentration (nM)** | **Size (bp)** | **Result** |
|---|---|---|---|---|---|---|
| 1 | Nail (Prediabetes and hypertension risk group) | SureSelect V6-Post (gDNA) | 29.8 | 103.5 | 443 | Pass |
| 2 | Whole blood (Prediabetes and hypertension risk group) | SureSelect V6-Post (gDNA) | 43.6 | 177.9 | 377 | Pass |
| 3 | Saliva (Prediabetes and hypertension risk group) | SureSelect V6-Post (gDNA) | 36 | 143.7 | 385 | Pass |
| 4 | Plasma (Prediabetes and hypertension risk group) | SureSelect V6-Post (cfDNA) | 25.7 | 122.9 | 322 | Pass |
| 5 | Nail (Diabetes patient group) | SureSelect V6-Post (gDNA) | 28 | 108 | 399 | Pass |

### [Example 2] NGS Sequencing

### NGS-based WES Analysis

Whole-exome sequencing (WES) is a method of analyzing only the sequences of the exome region, excluding non-coding regions; it can be seen as an example of Target Sequencing in that it targets specific regions; its primary purpose is to capture and amplify target regions and then search for specific variants through comparison with other samples. Compared to Whole Genome Sequencing (WGS), which analyzes the entire genome sequence, WES is easier to analyze due to its smaller data volume; furthermore, since most variants known to be associated with diseases occur in exon regions, WES analysis was conducted using NGS-based sequencing at a depth of 200X to 300X, which is an effective method in terms of time and analysis costs.

Compared to panel-based NGS analysis, which typically targets 10~120 types of gene mutations at a depth of 1,000X~10,000X or more for cancer diagnosis, performing sequencing at a lower depth of 200X~300X may lead to lower selection accuracy for variant genes, potentially making it insufficient for use in diagnostic fields. Therefore, while utilizing WES analysis capable of identifying more than 20,000 variants, an important algorithm used for identifying mutations (variants) by analyzing genomic data was developed and utilized for analysis to ensure improved variant detection performance in terms of sensitivity and accuracy aiming to reduce false positives and achieve 100% detection of missed variants. Analysis results were derived by comparing and double-verifying this with the Mutect2 variant caller, which is widely used by the majority of relevant researchers and companies domestically and abroad.

The developed variant caller algorithm is established through the following process, and the filtering step was improved based on a linear regression model obtained from simulation data to ensure high accuracy; in particular, it is a method optimized to maintain high sensitivity even in samples with low variant allele frequency.
1. Loading Python Packages
   A. import argparse
      ■ A package for receiving input arguments.
   B. import numpy as np
      ■ A package required for calculating depth during the vcf creation stage.
   C. import pandas as pd
      ■ A package for storing and processing variant information as DataFrames.
   D. import pickle
      ■ A package required for loading and writing dictionaries (used for result filtering).
   E. import pysam
      ■ A package used for loading read information from bam files.
   F. import random
      ■ A package used for encrypting variant patterns.
   G. import re
      ■ A package for manipulating strings (used to extract variant information from cigarstrings and md tags).
   H. import scipy.stats as stats
      ■ A package for scientific computing (used for filtering strand bias).
2. Functions
   A. read info
      ■ Loads the positional information and sequences of reads contained in the md tags, cigarstrings, and bamfiles, and the corresponding reference genome.
      ■ Extracts positional information where snvs and deletions occurred using the re package. - cnt(list)
      ■ Identifies the specific types of bases where snvs and deletions occurred using the re package. - seq(list)
      ■ Trims soft clips from the read sequences and modifies the cigarstrings accordingly. - cigar(string)
         (1) A soft clip indicates a portion (primarily at both ends) that does not match when a read is aligned to the reference genome, mostly corresponding to adapter or molecular barcode sequences.
      ■ Stores the read sequence as a variable. - read _seq(string)
         (1) Categorizes cases where soft clips exist on the left, right, or both sides, and preserves only the purely mapped read sequence based on the deletion positions.
      ■ Loads the reference genome sequence at the position where the read is mapped. - ref_seq(string)
      ■ Stores the genomic position of the read as a variable. - block_start(int)
   B. prep_indel
      ■ A preparation process for detecting indels; uses the cigar.
      ■ Identifies patterns of M (match), D (deletion), and I (insertion) from the cigar. - indel_pattern(list)
      ■ Identifies positional information for M (match), D (deletion), and I (insertion) from the cigar. - indel_iter(list)
      ■ For example, if the cigar is 52M2I28M2D63M, it indicates that 2 insertions occur after 52 base matches, 2 bases are deleted after 28 matches, and the remaining 63 bases match. In this case, indel_pattern becomes ['M', 'I', 'M', 'D', 'M'] and indel_iter becomes [52, 2, 28, 2, 63].
      ■ Using a for loop, the relative positions of deletions and insertions within the read and the number of inserted/deleted bases are identified by moving the positions of the read and the reference along the indel patterns. del_pos_list(list), ins_pos_list(list), del_size(list), ins_size(list)
      ■ An explanation of the above example is as follows:
         (1) Since the first indel element of the indel_pattern is I (insertion), ref_pos is added by the matching number (52) before the insertion. Since it is the first indel, alt_pos is also the same. ins_pos_list is based on alt_pos. The insertion position obtained in this way is added to ins_pos_list. That is, ref_pos : 52, alt_pos : 52, ins_pos_list : [52].
         (2) The second indel element of the indel_pattern is D (deletion), and ref_pos is added by the matching number (28) after the existing ref_pos (Step 1: 52). At this time, for alt_pos (Step 1: 52), since two bases were inserted in the previous Step 1, the inserted 2 is added to the existing alt_pos, and then the matching number (28) is added. del_pos_list is based on ref_pos. That is, ref_pos : 80, alt_pos : 82, del_pos_list: [80].
         (3) Summarizing the above results,
            (A) del_pos_list = [80]
            (B) del_size = [2]
            (C) ins_pos_list = [52]
            (D) ins_size = [2]
   C. call_indel
      ■ Using del_pos_list/del_size and ins_pos_list/ins_size obtained from the prep_indel process, the genomic position of the indel and the deleted or inserted base sequences are stored as a dictionary variable. - key: variant type (indel), value: genomic position, ref, alt (list)
         (1) Insertion : As inserted bases, the base information can be found in read_seq. The inserted base sequence is obtained using the insertion position and insertion size in read_seq.
         (2) Deletion : As deleted bases, the base information can be found in ref_seq. The deleted base sequence is obtained using the deletion position and deletion size in ref_seq.
   D. call_snv
      ■ As a function to detect snvs, the cnt and seq lists obtained from the md tag are used.
      ■ Since the position information of snvs varies depending on the presence of indels, the output variables of call_indel are used as input variables.
      ■ Similar to prep_indel, the read and reference sequences are moved from left to right to find the position corresponding to cnt. - Using a for loop.
      ■ 0 is designated as the initial value for ref_pos (reference) and alt_pos (read). (Initial starting point)
      ■ From cnt = [34] and seq = ['C'], it can be identified that the 34th C base was substituted with another base and that the total number of snvs is one.
      ■ Moving 34 bases from the left in ref_seq results in C, and moving 34 bases in read_seq results in G. That is, the 35th C has been changed to G.
      ■ If one of the cnt elements starts with ^, it means a deletion occurred and it is not an snv; therefore, only ref_pos increases (because it was deleted) and alt_pos does not change.
      ■ Conversely, if an insertion occurs, the information is not in cnt but in ins_pos_list (referred to as alt_pos_list in this function), which is an output of prep _indel; in this case, ref_pos does not change and only alt_pos increases. alt_pos retrieves position information from cnt, but since cnt does not contain insertion information, calculations must consider the relative positions of the substitution and insertion of the corresponding cnt to prevent duplicate insertion information when calculating with reference to ins_pos_list. That is, alt_pos increases by the number of inserted bases only when the insertion appears before the substitution. At this time, the insertion position is stored in a variable (last_ins) to prevent alt_pos from increasing redundantly.
      ■ The information obtained from this is stored in the var_dict dictionary, similar to call_indel. key: variant type (snv), value: genomic position, ref, alt (list)
   E. make_res
      ■ The process of converting the variant information stored in the dictionary into lists by dividing them into forward read/reverse reads is performed.
      ■ Each element of this list contains a single line string, which sequentially represents chromosome, genomic_position, ref, and alt.
      ■ After converting this list into a dataframe using pandas, the identical variants are counted.
      ■ The depth of each variant position is calculated using the count function of pysam.
      ■ Two dataframes, which store the variants of the forward/reverse reads respectively, are produced as the final output.
   F. filter_output
      ■ It is a process of filtering each variant based on its own criteria.
      ■ low_vaf
         (1) Create a fastq without variant information using art_illumina, a fastq simulator. All variants generated at this time are treated as errors. A linear regression model is created using the maximum value of the error count for each depth.
         (2) If a variant from make_res is lower than the value of this model, it falls into the error category and will be tagged with a low_vaf flag in the subsequent make_vcf process.
      ■ low_depth
         (1) The average coverage of whole exome sequencing is 50, and variants with a depth of 40 or less will be tagged with this flag. This value is subject to change depending on experimental results.
      ■ strand_bias
         (1) Strand bias refers to a phenomenon where variants are concentrated in either forward or reverse reads.
         (2) The Broad Institute recommends calculating the FisherStrand probability, and the same criteria were applied to this algorithm.
         (3) By retrieving depth and count information from the final dataframe, [[depth1, depth2], [read1, read2]] is stored in a variable.
         (4) The p-value is obtained using the scipy.stats package.
         (5) The Phred score is obtained.
         (6) If the Phred score is less than 60, it is determined that there is no strand bias.
      ■ Germline/panel of normal filter
         (1) The panel of normal vcf (variants appearing in normal groups) and germline variants provided by the Broad Institute are excluded from the results.
   G. find_pattern
      ■ In the process of detecting variants, if multiple variants are found in a single read, they are stored separately as outputs.
      ■ Using getrandbits from the random package, a unique ID (a 32-bit value) is assigned to each variant. Identical variants have the same ID. The ID assigned to each variant is stored in a dictionary named patterninfo.
      ■ If three variants exist in a single read, they are stored in a pattern (list) in the form of [ID1, ID2, ID3].
      ■ Once variant detection is complete, identical patterns within the pattern are converted into a dataframe and counted.
      ■ The variant information matching the IDs is retrieved from patterninfo.
   H. make_vcf
      ■ The process of creating a VCF from the result dataframe.
      ■ After loading a template VCF, information is retrieved from the dataframe to fill each column.
      ■ It consists of '#CHROM', 'POS', 'ID', 'REF', 'ALT', 'QUAL', 'FILTER', 'INFO', 'FORMAT', and the sample name.

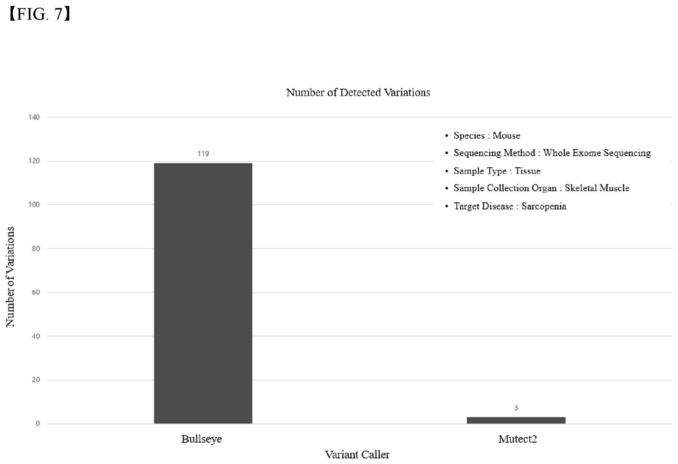

To compare the in-house caller **(Bullseye)** developed in the method described above with a widely used Variant caller (Mutect2), variants were detected from preprocessed bam files using the (in-house caller) to obtain vcf files; subsequently, annotation was performed on the VcF files using ensembl's vep, and the Vep outputs were integrated and processed with the vcf information. When analysis results were derived by filtering based on specific disease-related genes, in the case of WES gene variant analysis related to sarcopenia, it was confirmed that approximately 40 times more disease-related variants were additionally detected when applying the developed in house caller algorithm compared to the existing variant caller (Mutect2), as shown in FIG. 7.

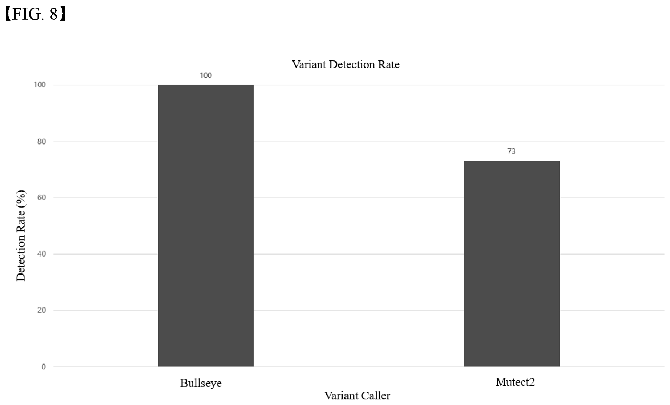

In addition, as a result of comparative analysis using a non-small cell lung cancer reference material (Seracare Tri-Level Tumor Mutation DNA Mix v2), as shown in Table 2 and FIG. 8 below, the in house caller detected all variants included in the reference material, whereas Mutect2 failed to detect two of the variants.

**[Table 2]**

| Legacy ID | Genomic mutation ID | Ge ne | AA mutation | CDS mutation | Genomic coordinates | Mut ect2 | Bulls eye |
|---|---|---|---|---|---|---|---|
| COSM 476 | COSV56056 643 | BR AF | p.V600E | c.1799T>A | chr7:14075333 6 | o | o |
| COSM 12378 | COSV51769 298 | EG FR | p.D770_N 771insG | c.2310_231 1insGGT | chr7:55181319 -55181320 | o | o |
| COSM 6225 | COSV51765 066 | EG FR | p.E746_A7 50del | c.2236_225 0del | chr7:55174773 -55174787 | x | o |
| COSM 6240 | COSV51765 492 | EG FR | p.T790M | c.2369C>T | chr7:55181378 | x | **o** |
| COSM 6224 | COSV51765 161 | EG FR | p.L858R | c.2573T>G | chr7:55191822 | o | o |
| COSM 521 | COSV55497 369 | KR AS | p.G12D | c.35G>A | chr12:2524535 0 | o | o |
| COSM 6530 | COSV52760 886 | TP 53 | p.C242Afs *5 | c.723del | chr17:7674241 | o | o |
| COSM 10662 | COSV52661 580 | TP 53 | p.R248Q | c.743G>A | chr17:7674220 | o | o |
| COSM 10648 | COSV52661 038 | TP 53 | p.R175H | c.524G>A | chr17:7675088 | o | o |
| COSM 10660 | COSV52660 980 | TP 53 | p.R273H | c.818G>A | chr17:7673802 | o | o |

Cases of false negatives have been reported several times in Mutect2, which is most representatively used and known for its good performance at low frequencies (Krøigård, A. B., Thomassen, M., Lænkholm, A.-V., Kruse, T. A., & Larsen, M. J. (2016). Evaluation of Nine Somatic Variant Callers for Detection of Somatic Mutations in Exome and targeted Deep Sequencing Data. PLOS ONE, 11(3), e0151664). That is, there are cases where clinically significant variants are not detected, and there may be a possibility of errors in the method of calculating the probability of a variant's presence using an internal probabilistic model of the detection algorithm.

The developed in house caller (Bullseye), when clinical samples and reference materials were comprehensively tested, was able to find variants even at low depth and frequency by using a method of identifying parts different from the reference genome sequence by reading all read information; as a result of the performance evaluation, it demonstrated sensitivity and accuracy 1.3 to 39.7 times or more superior to Mutect2.

### [Example 3] Genetic Analysis Test

### Genetic analysis test for patients with chronic diseases and cancer

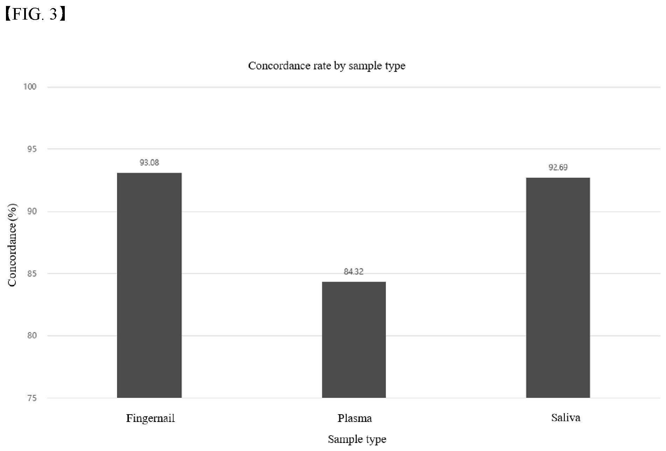

Genetic analysis tests based on nail specimens were conducted primarily for patients with chronic diseases and cancer. For primary verification, comparative analysis results of hereditary and non-hereditary disease markers related to diabetes were derived. In the observation results of genetic mutations related to diabetes, hypertension, and hyperlipidemia based on the germline mutations of a sample donor with borderline diabetes and hypertension, when comparing the detection from nail, plasma, and saliva samples to confirm the concordance rate of mutations detected against whole blood as a reference, it was confirmed that the nail samples exhibited similar or the highest detection patterns, as shown in FIG. 3. The above results were calculated as a percentage value by dividing the number of mutations commonly detected in each sample and whole blood by the total number of detected mutations.

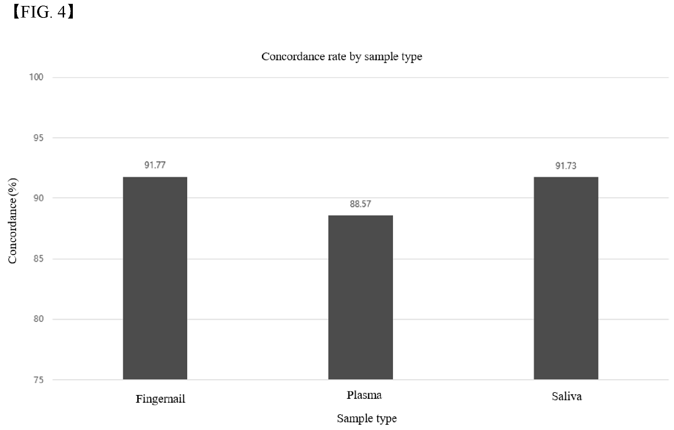

In the observation results of genetic mutations related to diabetes, hypertension, and hyperlipidemia based on the somatic mutations of a sample donor with borderline diabetes and hypertension, when comparing the detection from nail, plasma, and saliva samples against whole blood as a reference, it was confirmed that the nail samples exhibited similar or the highest detection patterns, as shown in FIG. 4. The above results were calculated as a percentage value by dividing the number of mutations commonly found in each sample and whole blood by the total number of detected mutations.

In the case of the diabetes patient group, when filtering only for diabetes using an annotation tool, mutations were identified in genes, such as ALAD, ANKRD17, ASH1L, ATXN3, AZIN1, CACNA1E, CASQ2, CDC25A, CEL, CEP104, CPZ, CRB1, CTNS, DMXL2, EPC1, ERBB3, FAM193A, FAM20C, GALC, INPP4B, IVD, MAF, MAP3K9, MBTD1, MECOM, MED13, MYH3, NCOA2, NRXN1, PACSIN1, PDE6B, PIAS2, PPP3CB, PRKRA, PUM2, QKI, ROCK1, RPL7, SPAG9, TFDP2, TNRC6A, TTK, VPS13A, YTHDF3, ZC3H15, ZNF326 and ZNRF3, which are reported to be associated with existing diabetes having pathogenicity, confirming the possibility of predicting and diagnosing diabetes even in gDNA from nails (Table 3). Common genetic mutations in metabolic diseases, such as obesity, hyperlipidemia (HL), type 2 diabetes mellitus (T2DM), and metabolic syndrome (MS), were detected, and the groups indicated in gray belong to risk prediction markers based on type 1 diabetes mellitus (T1DM).

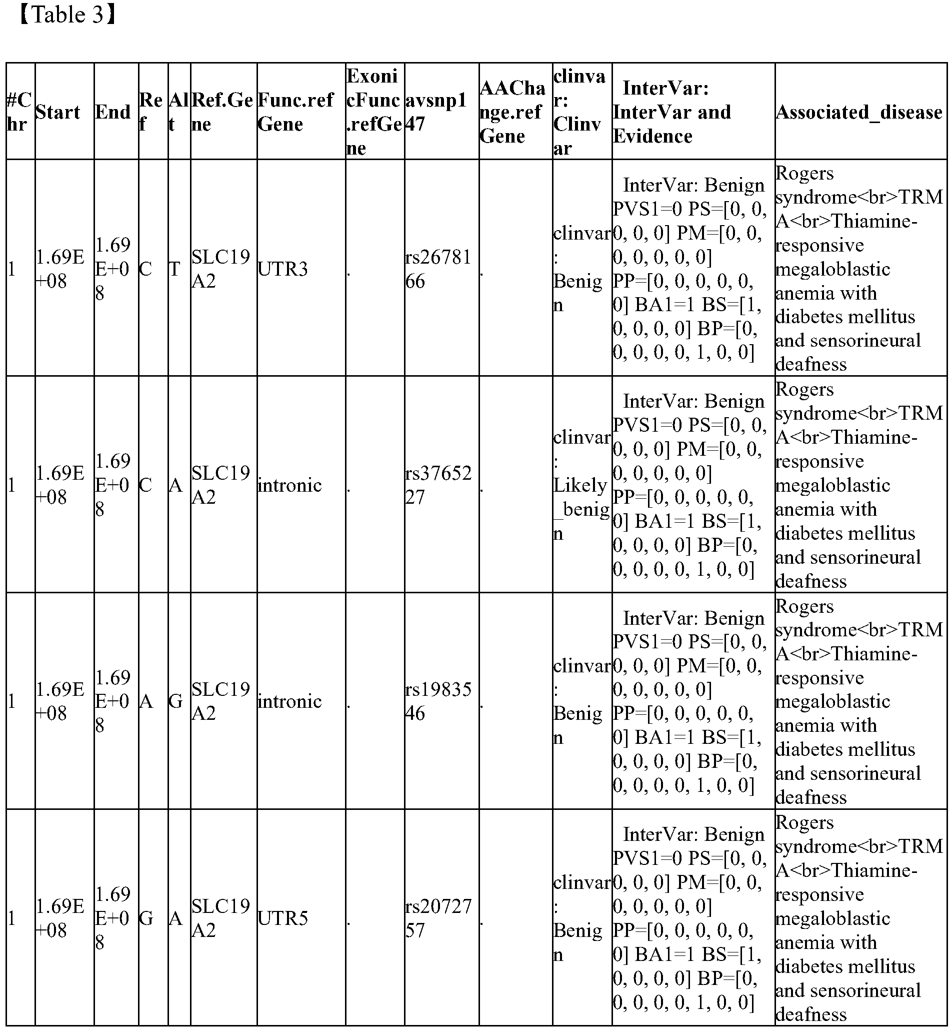

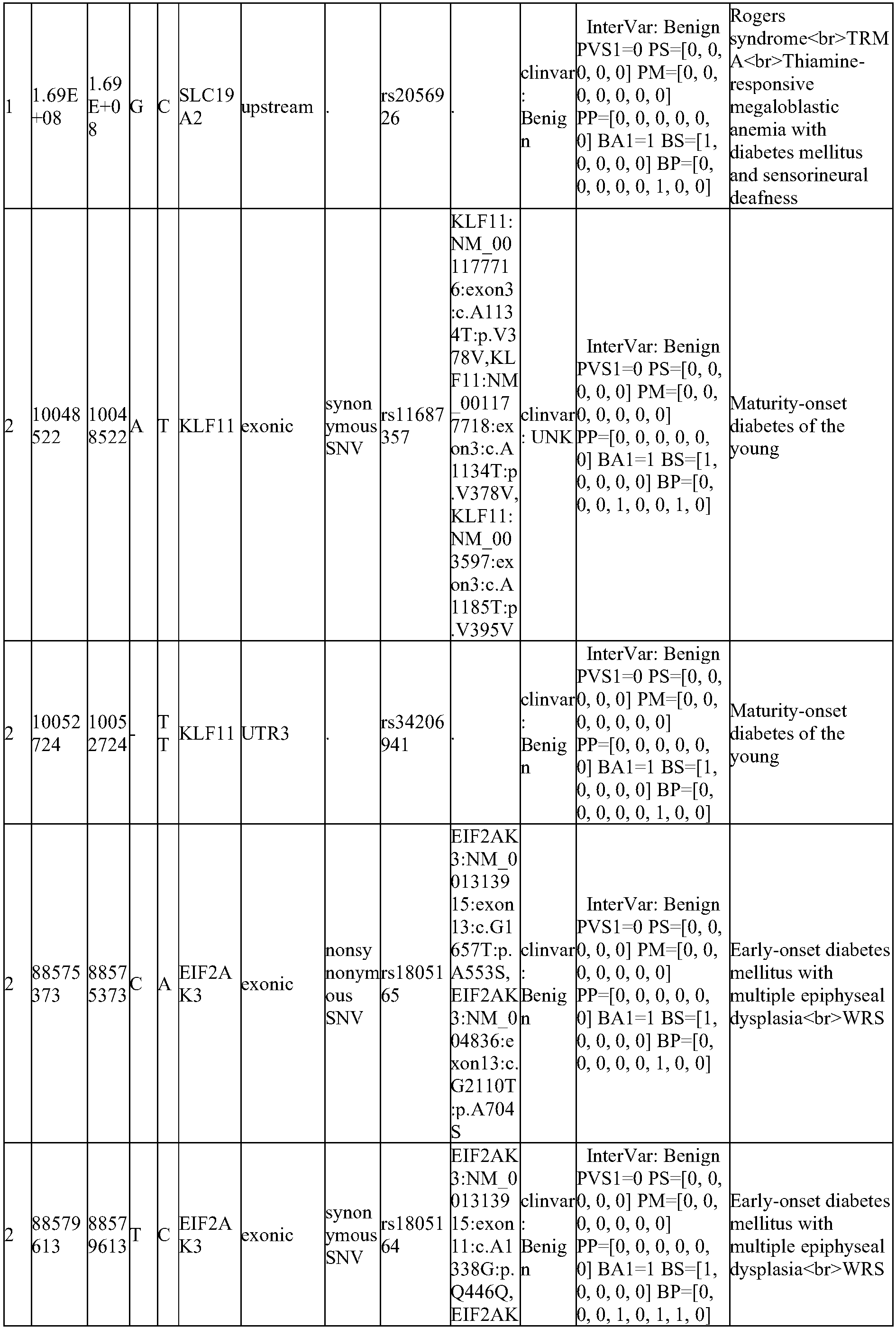

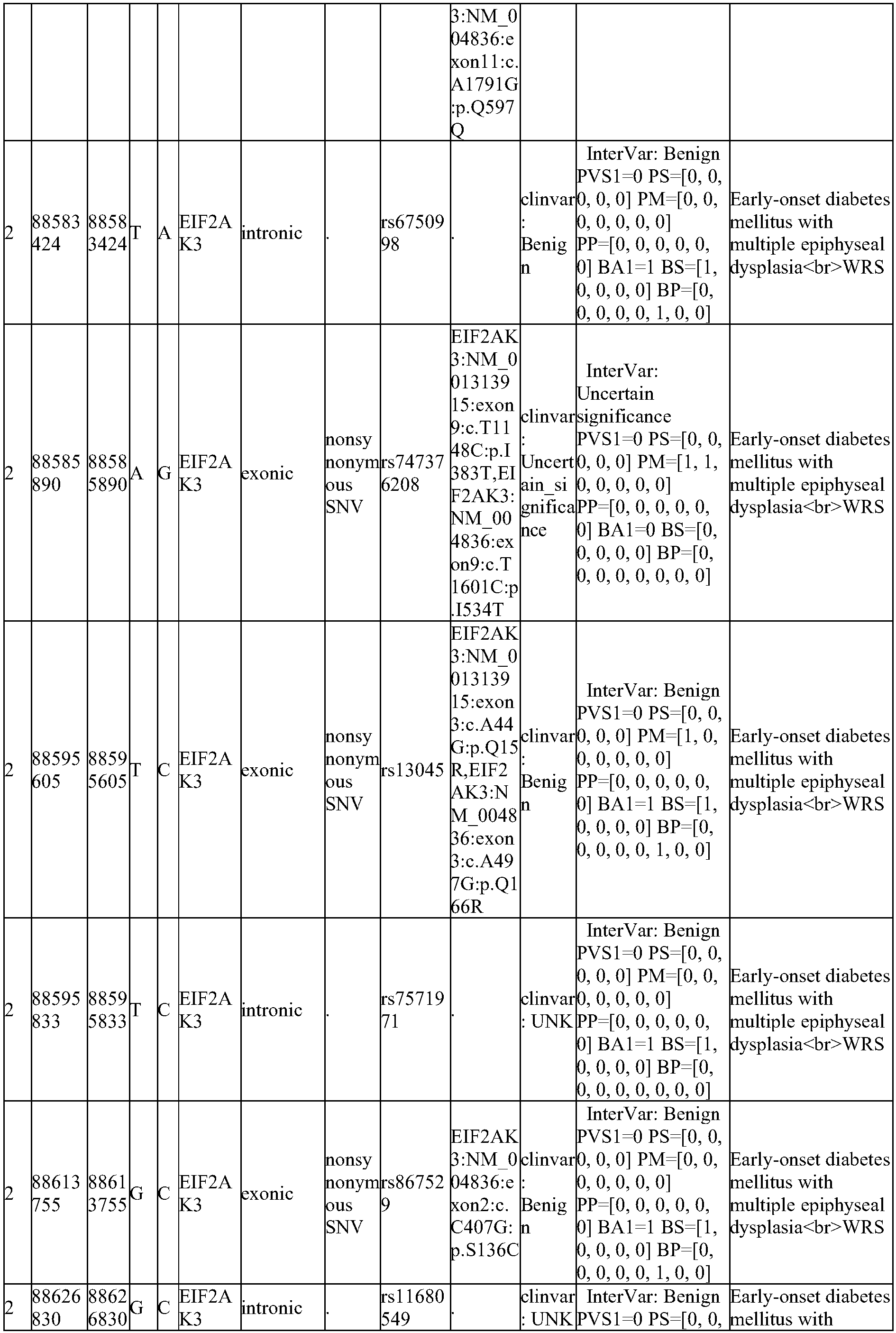

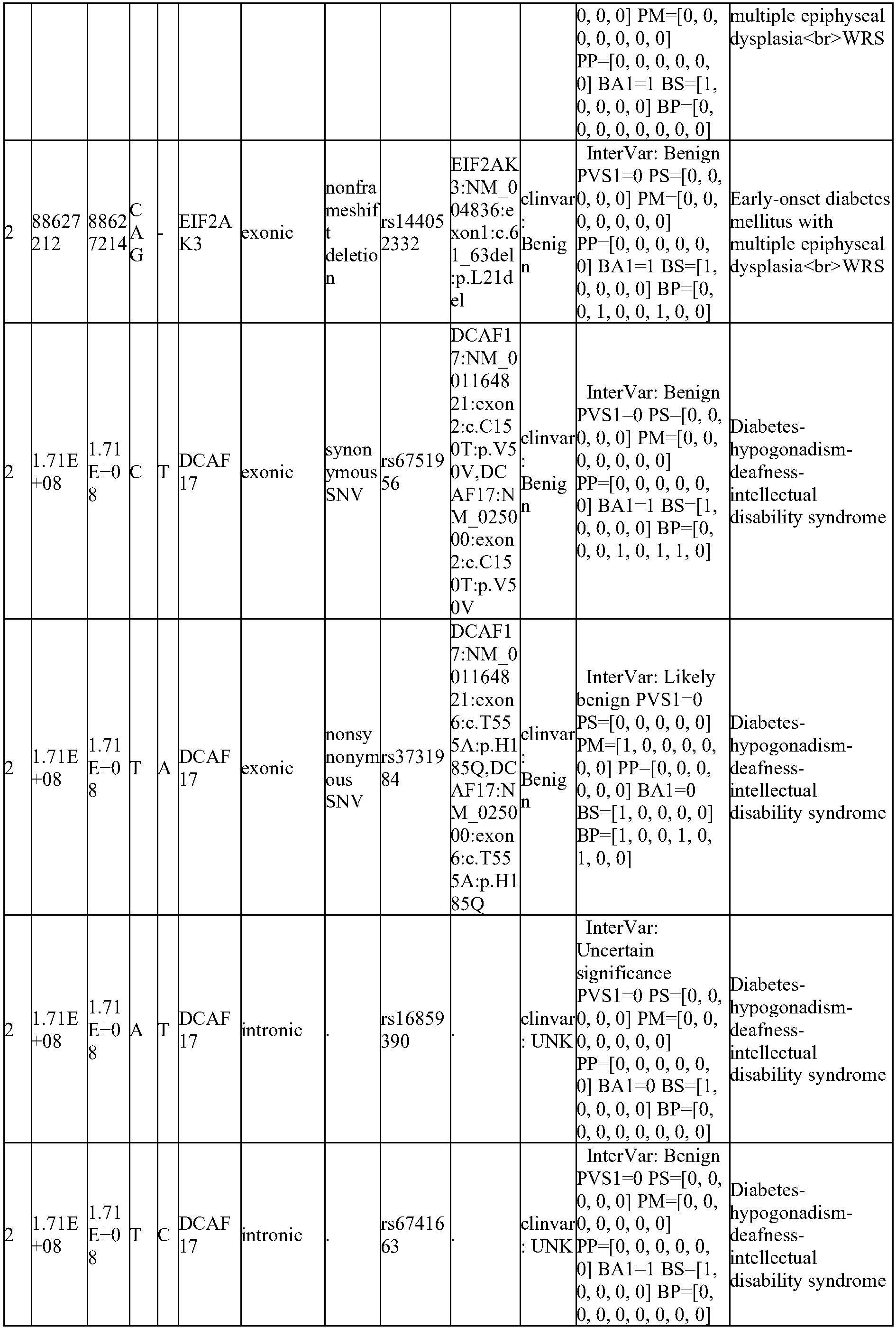

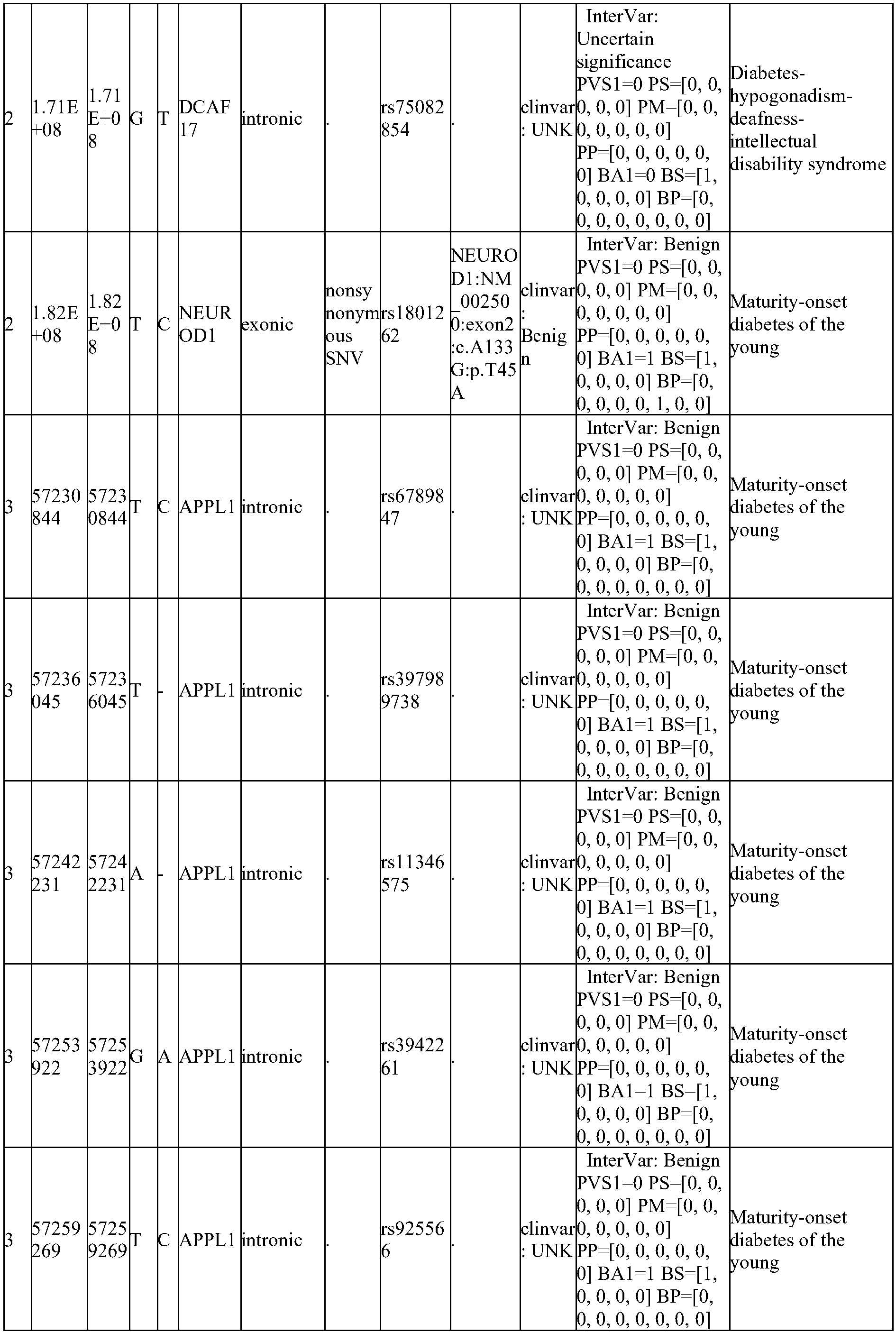

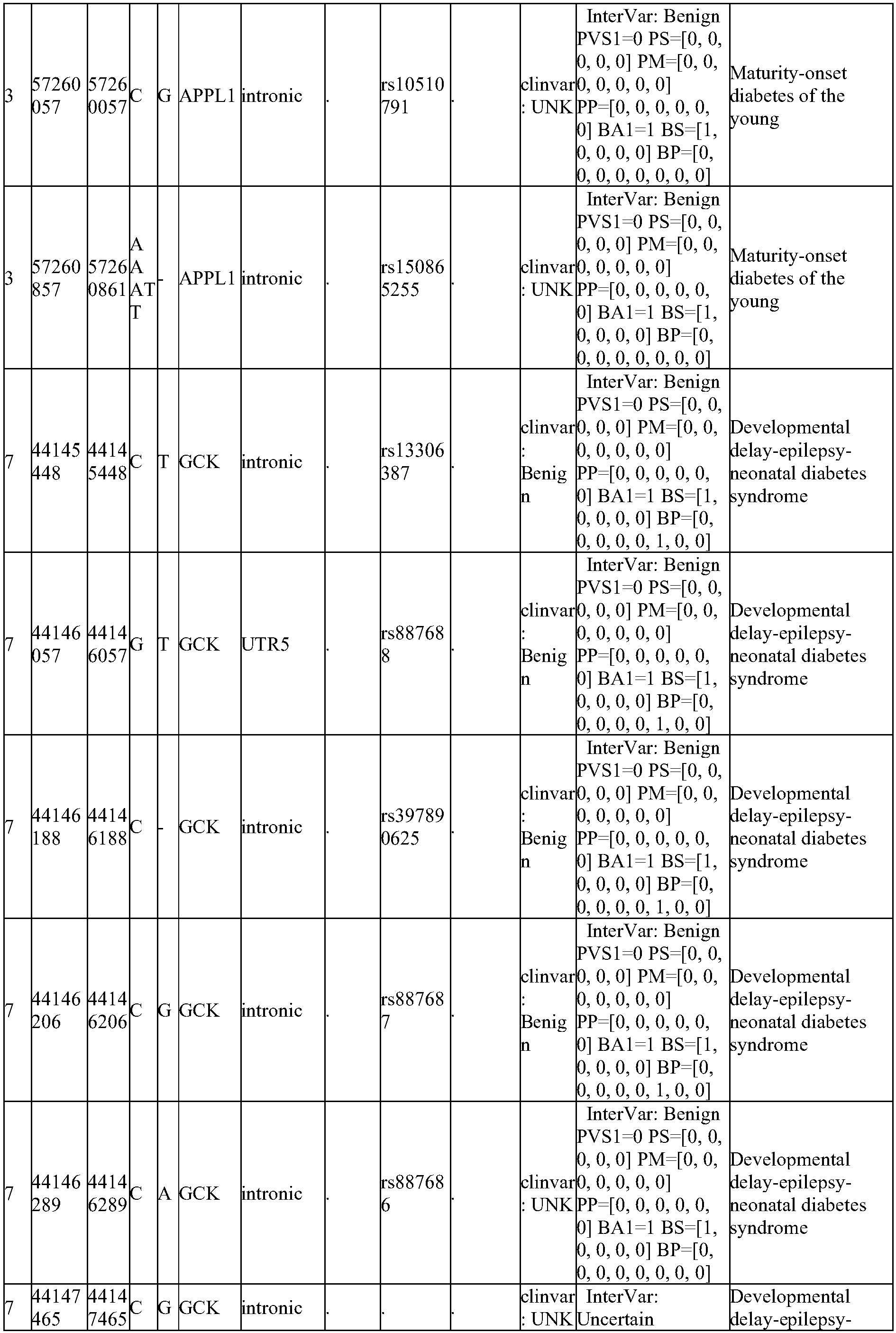

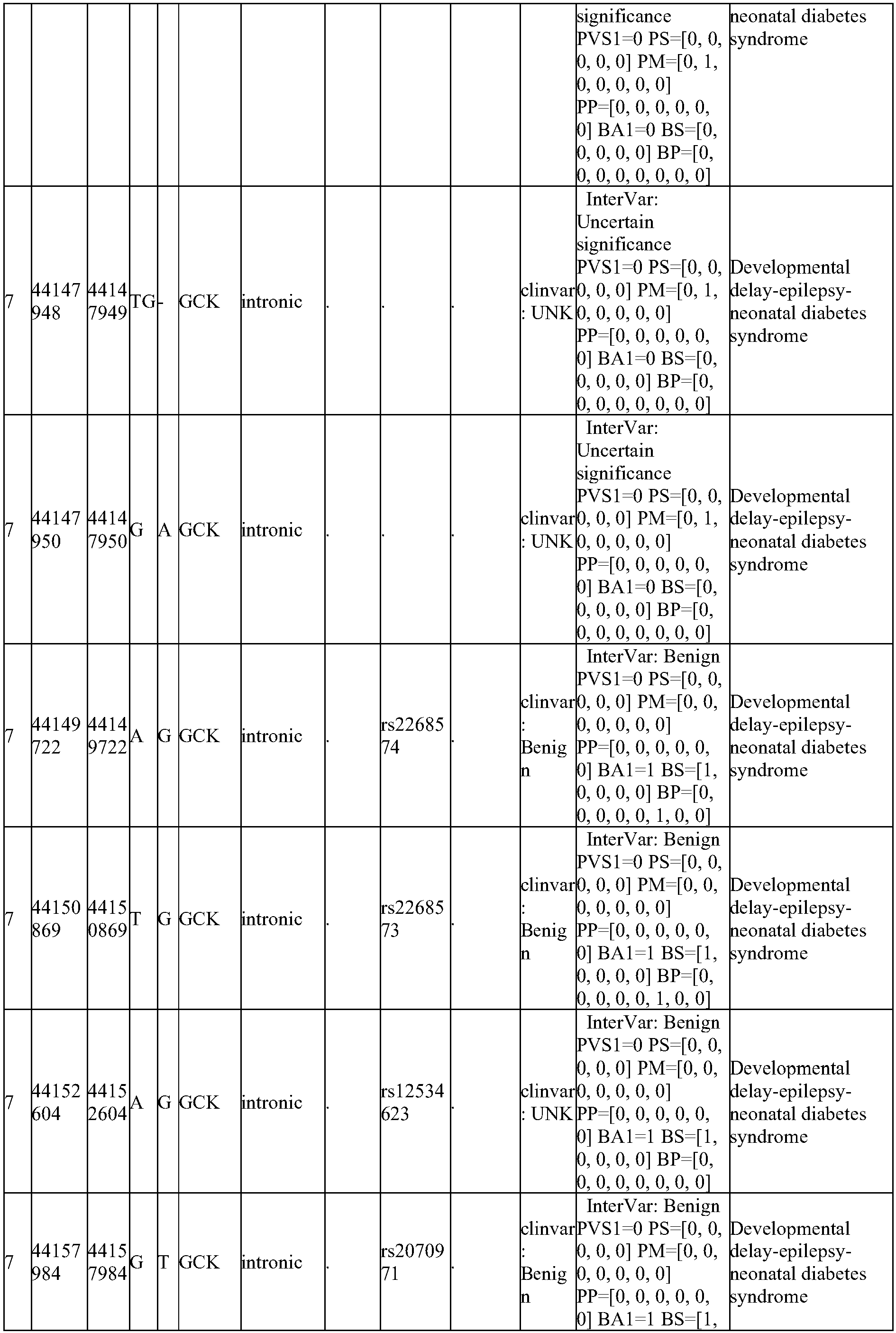

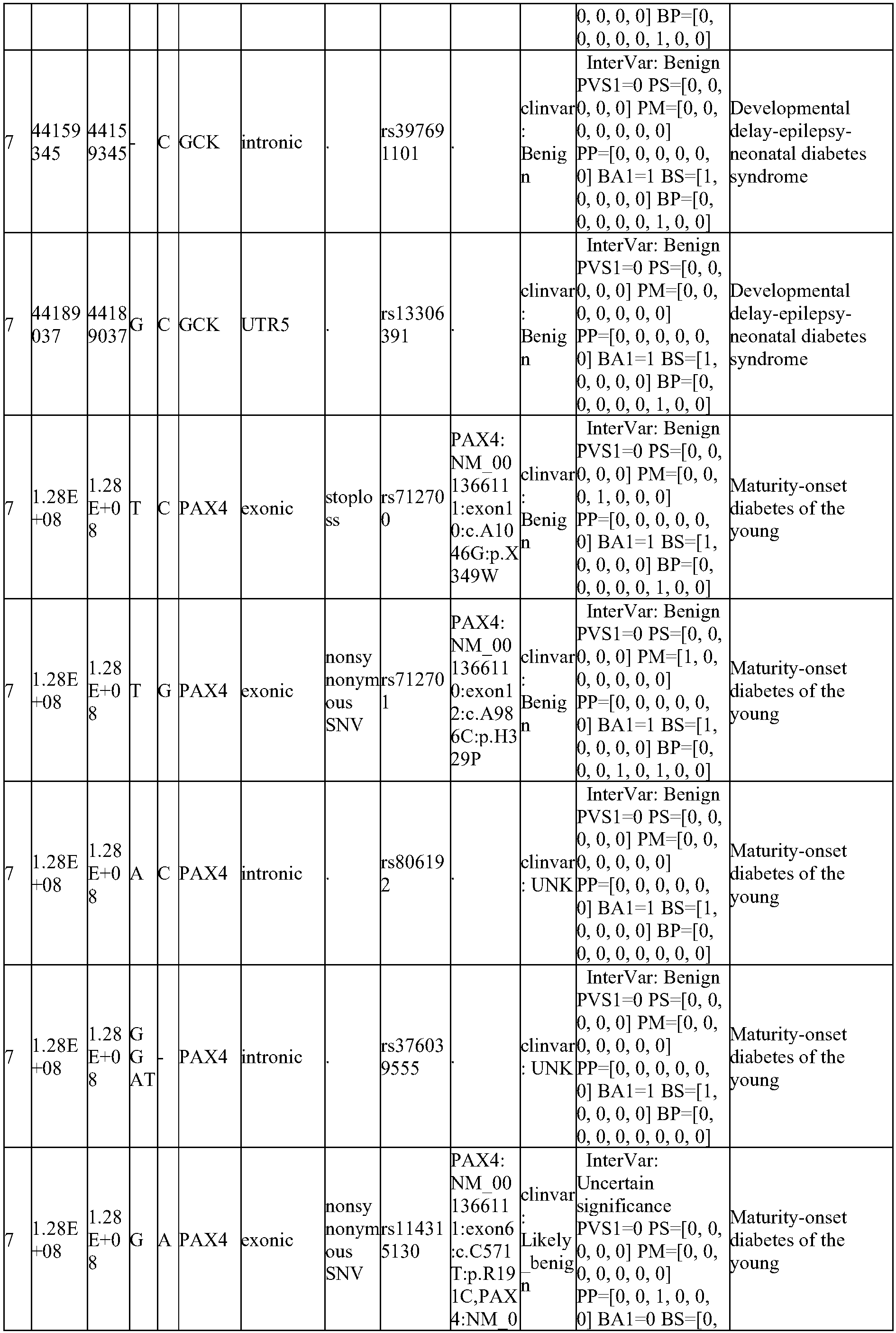

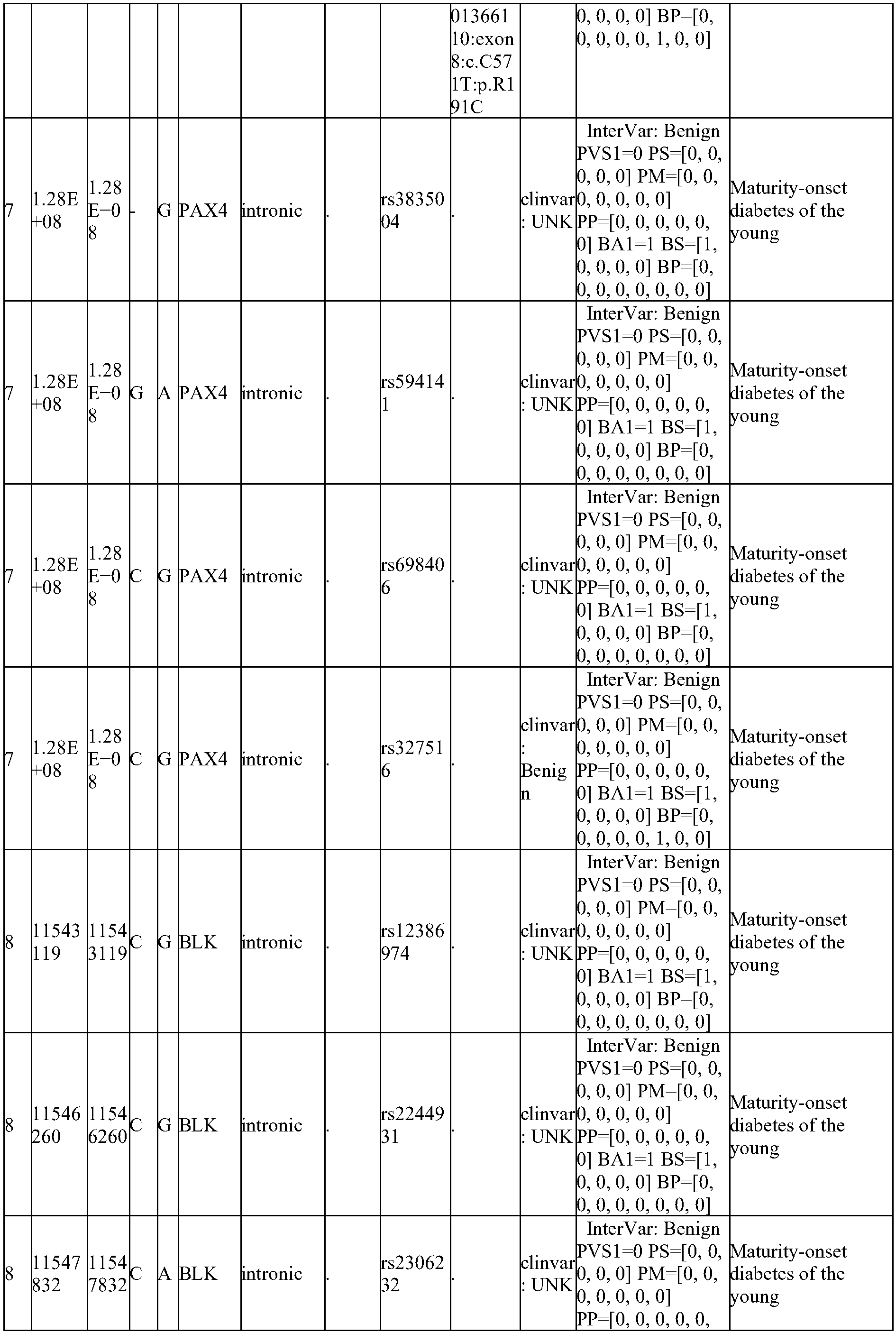

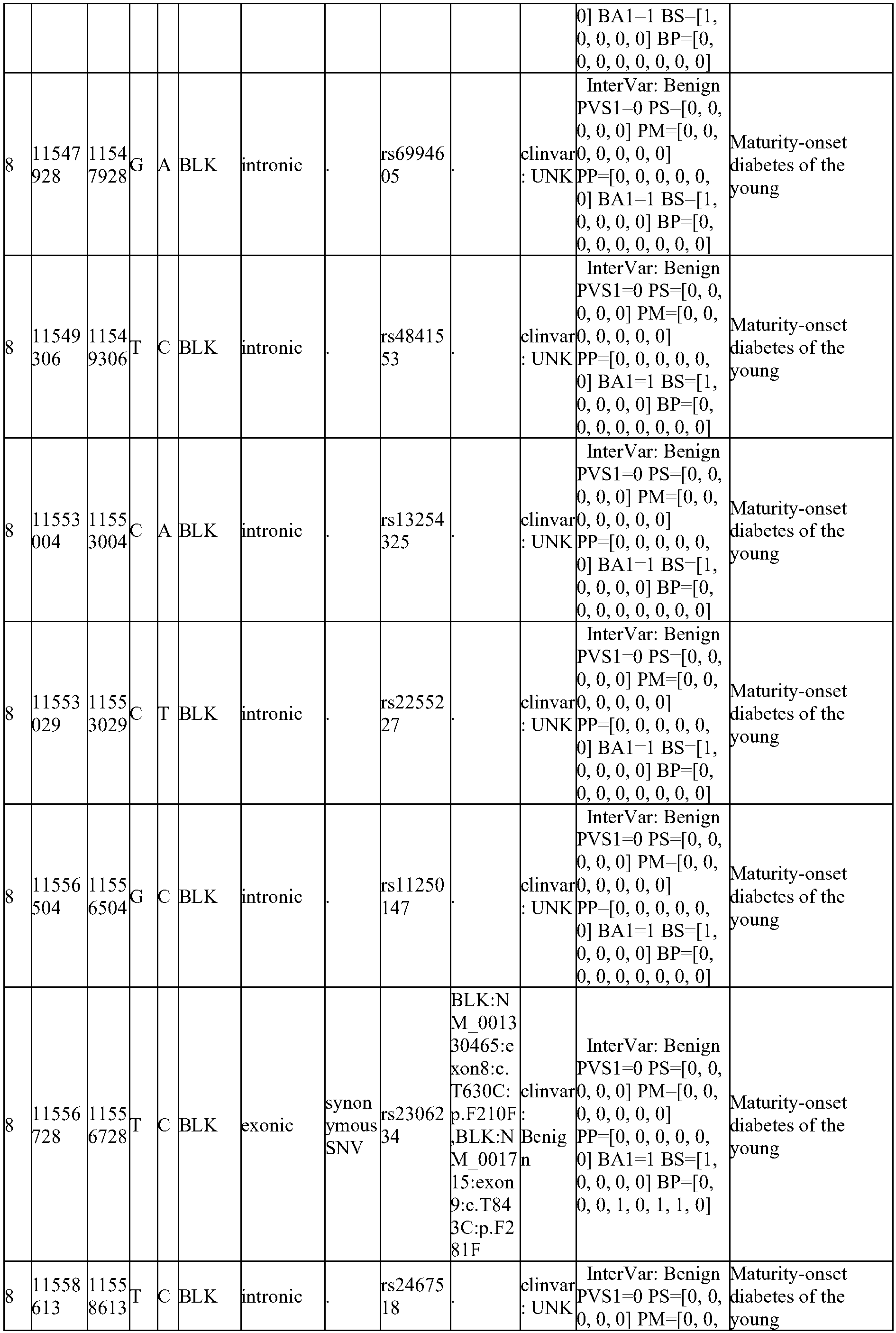

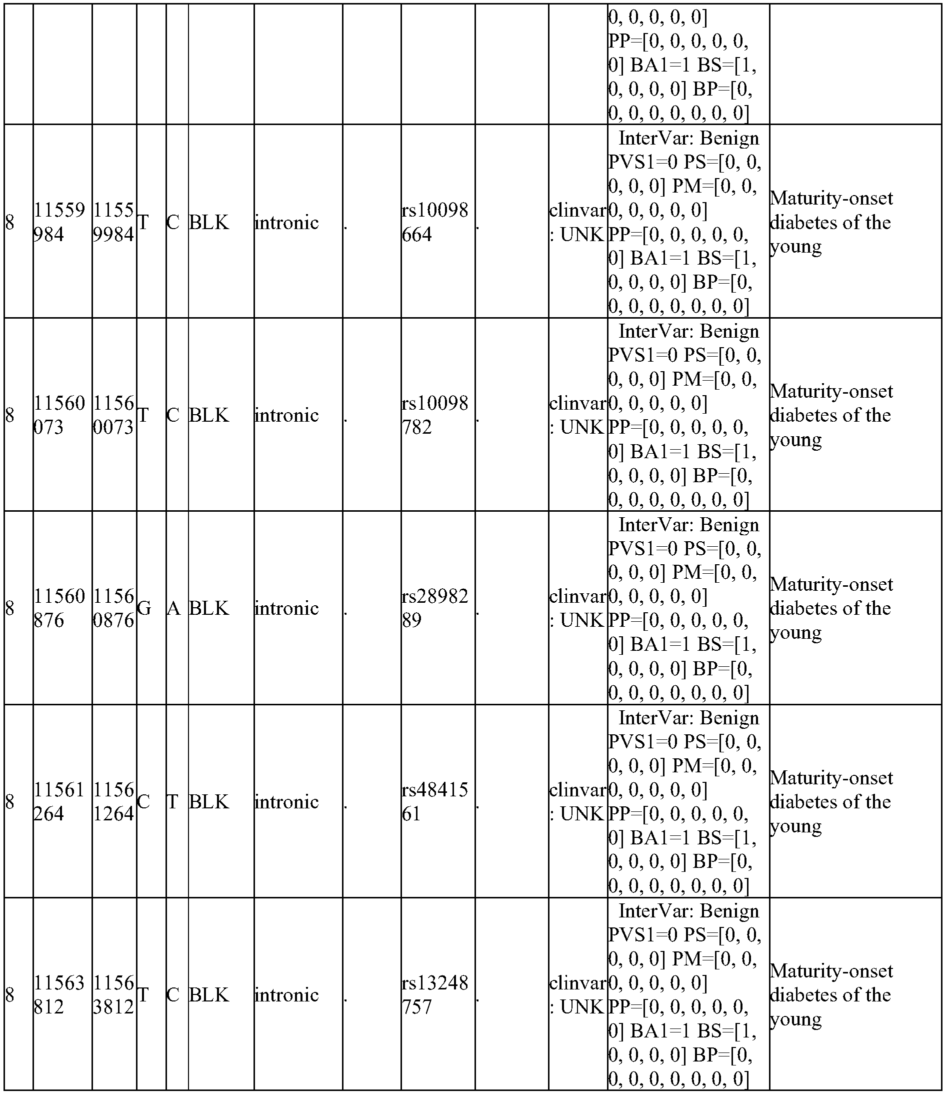

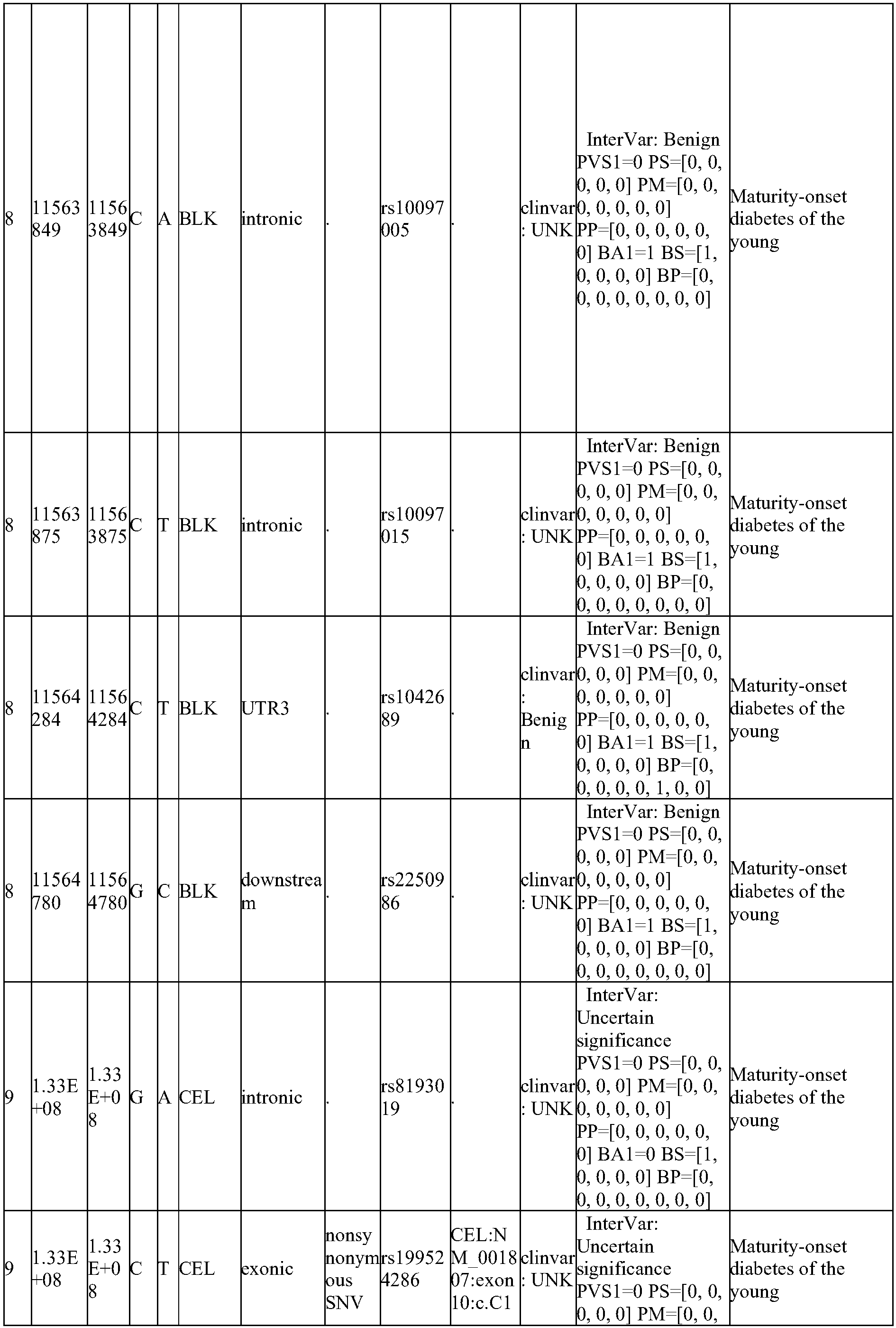

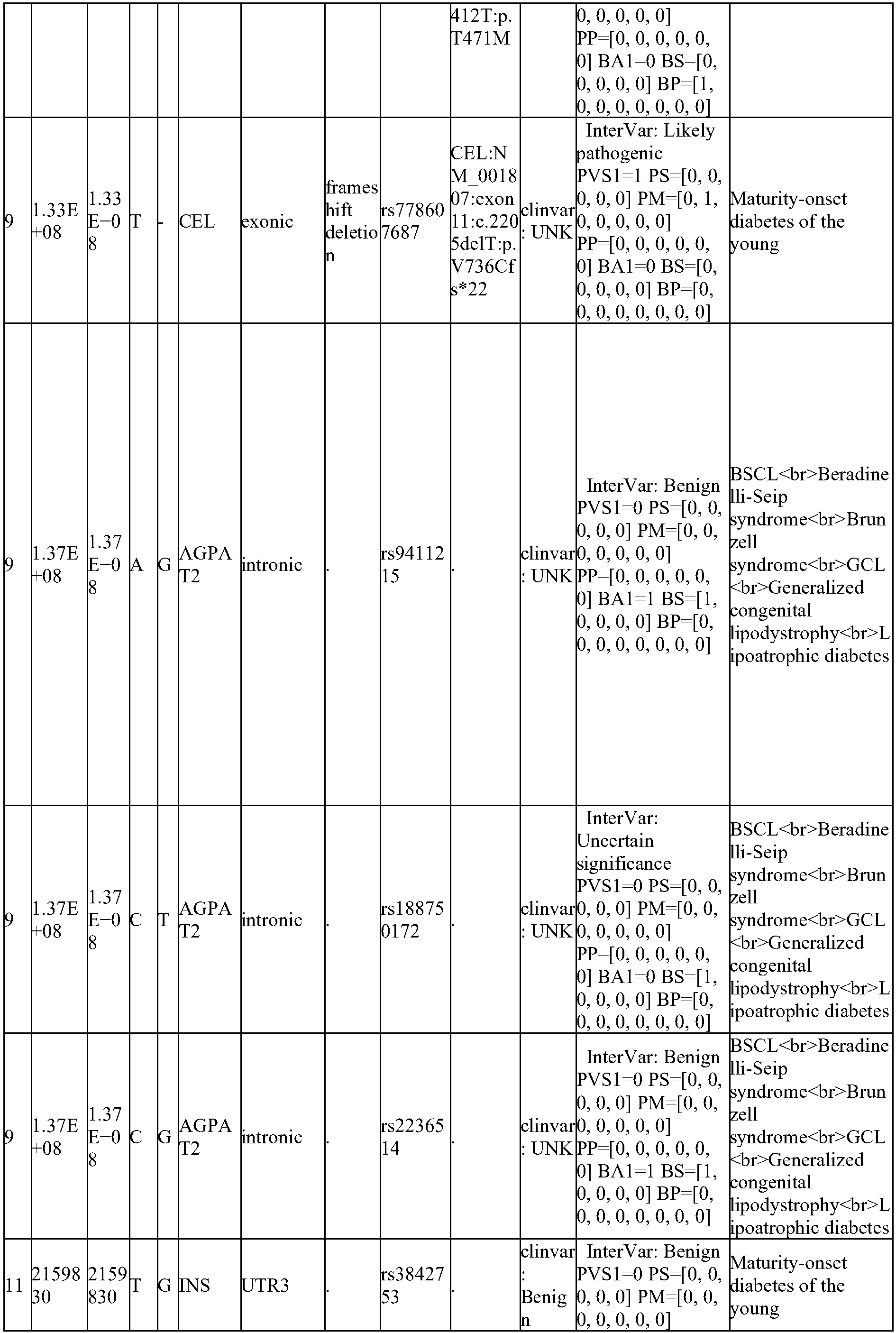

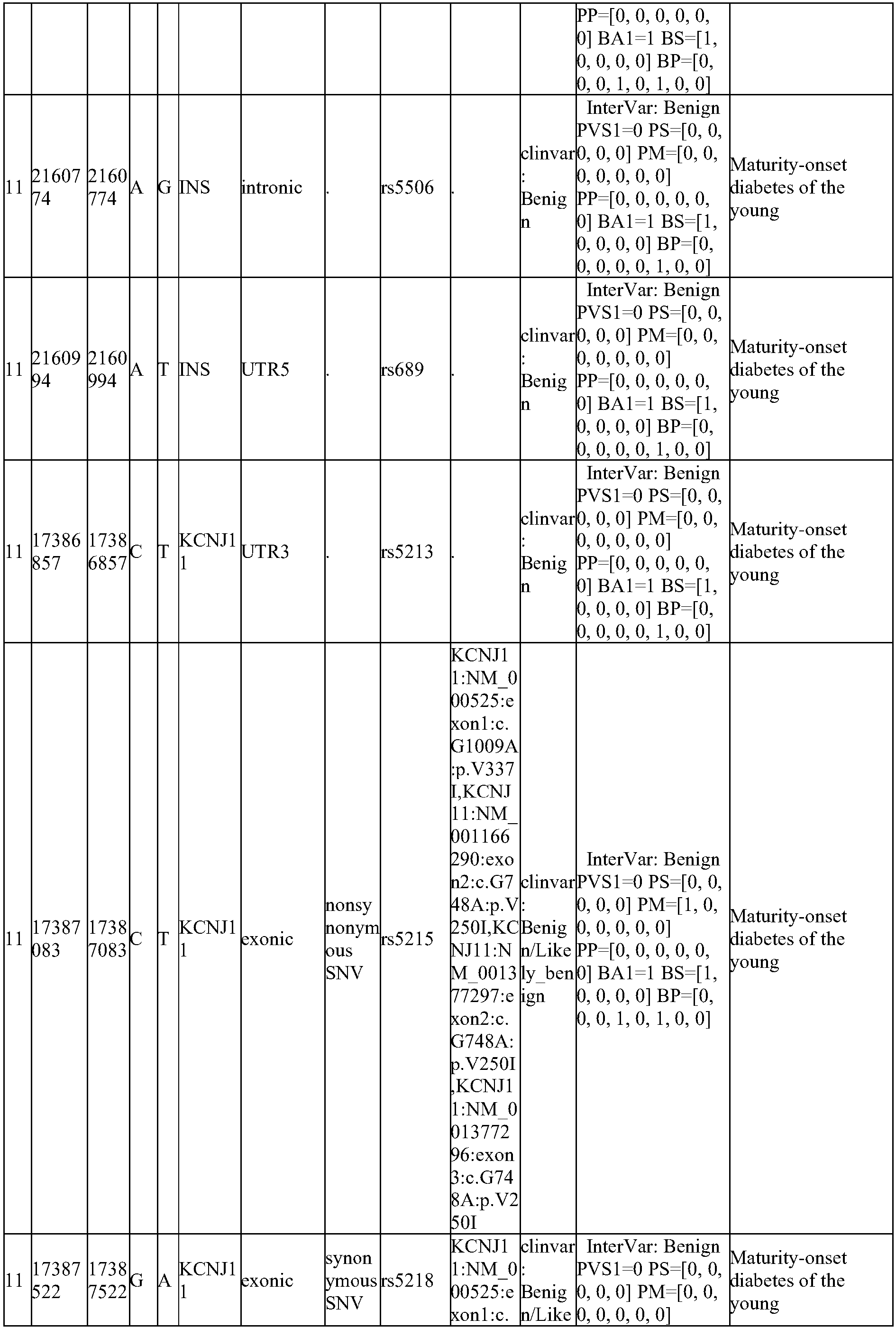

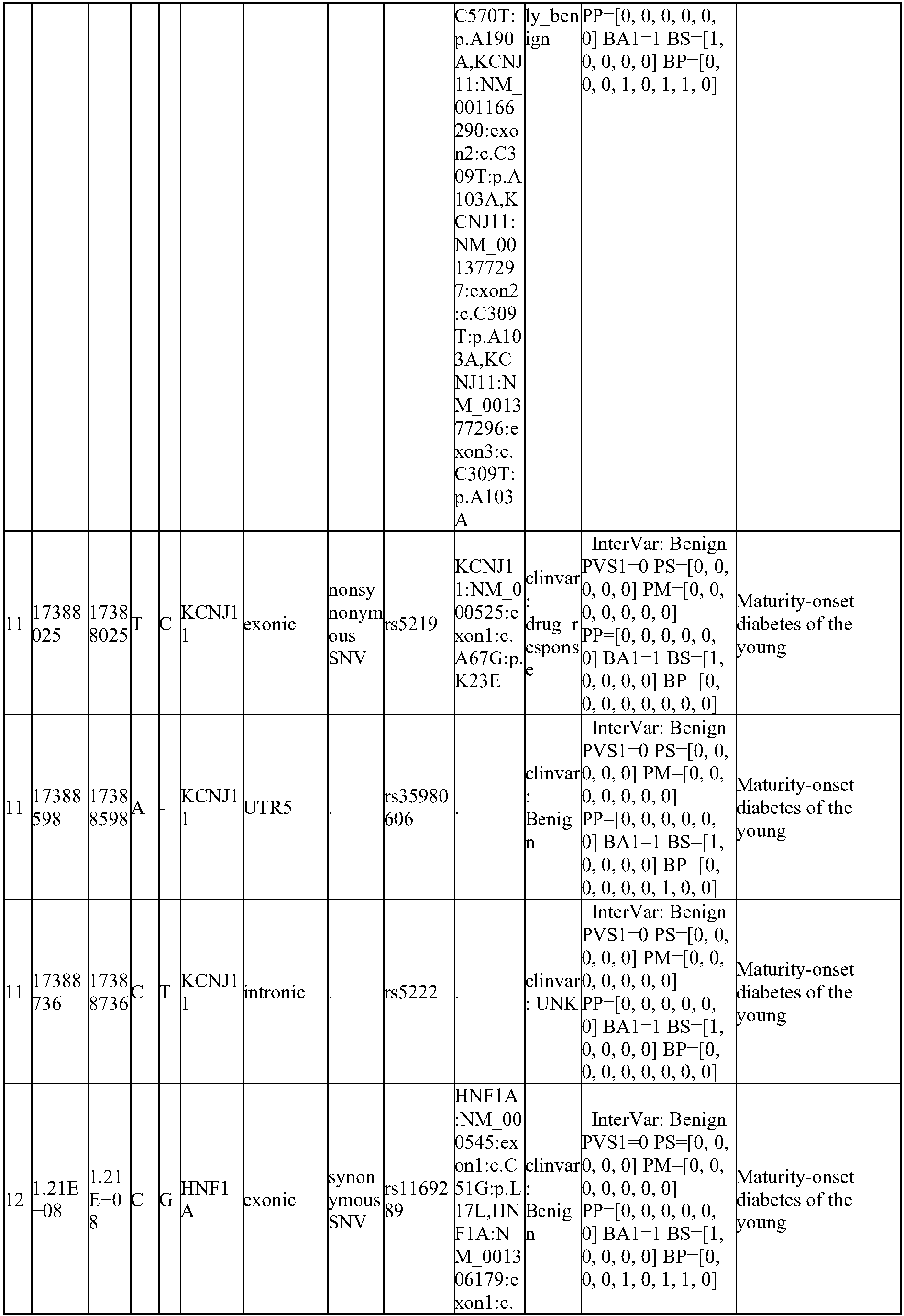

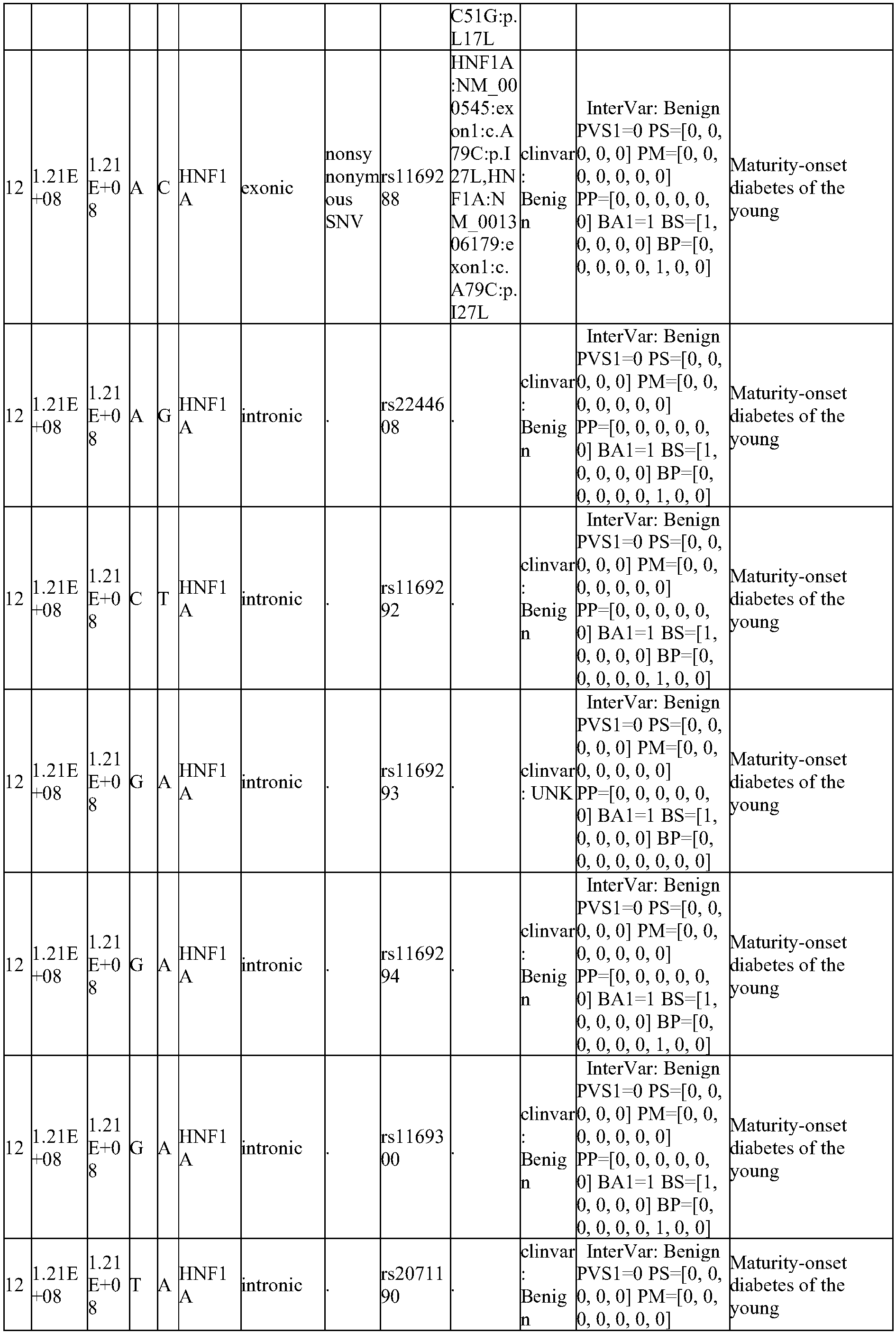

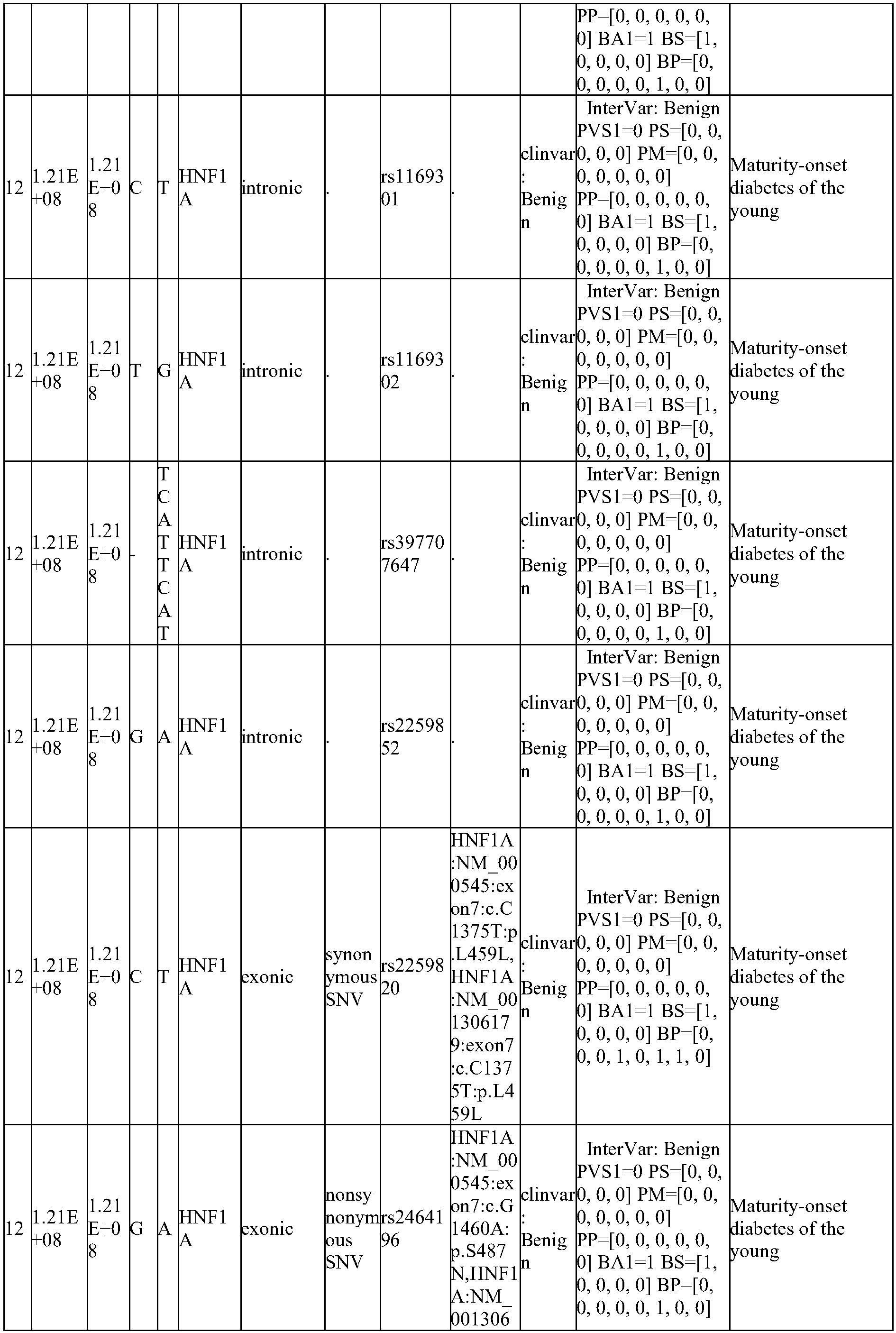

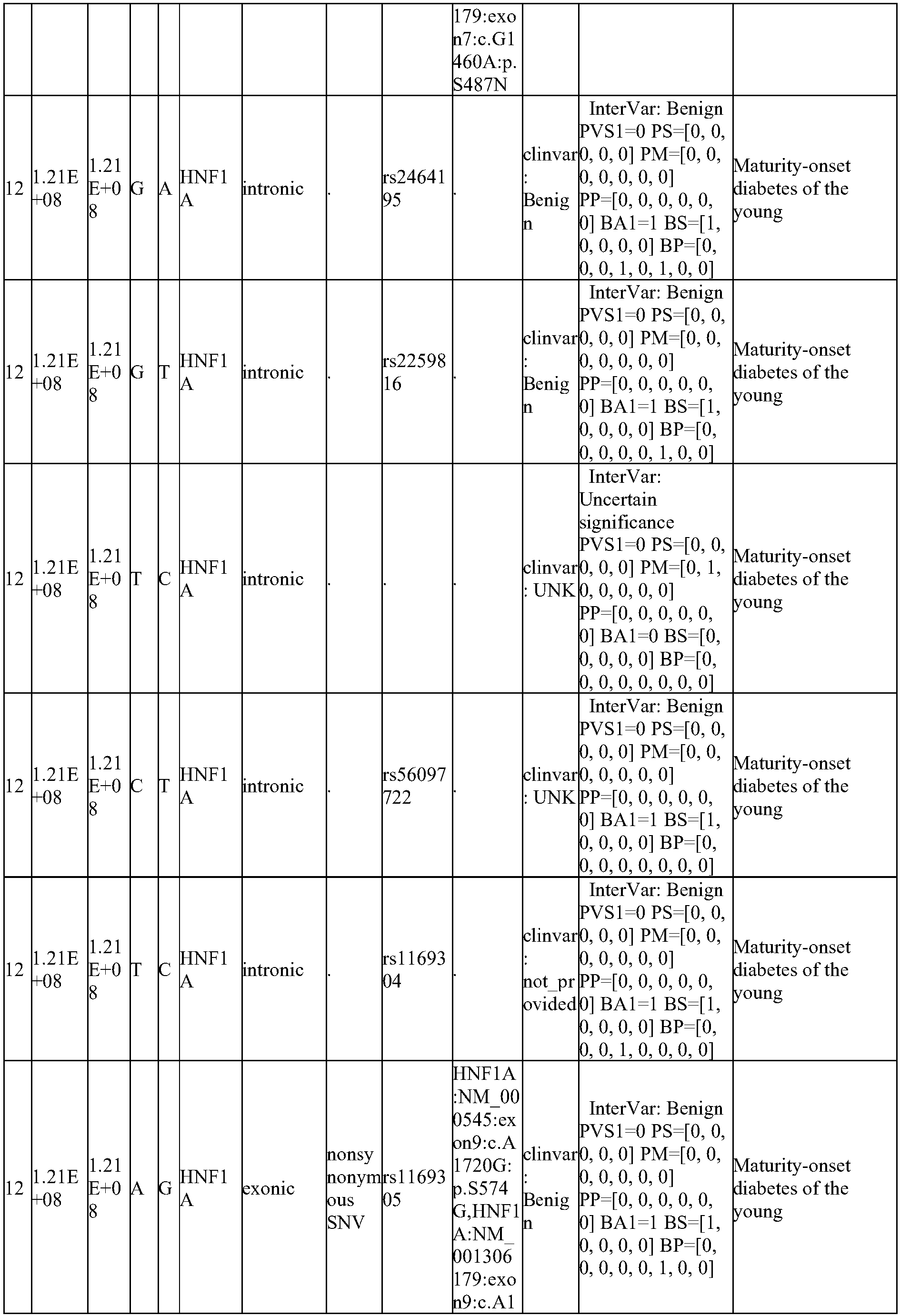

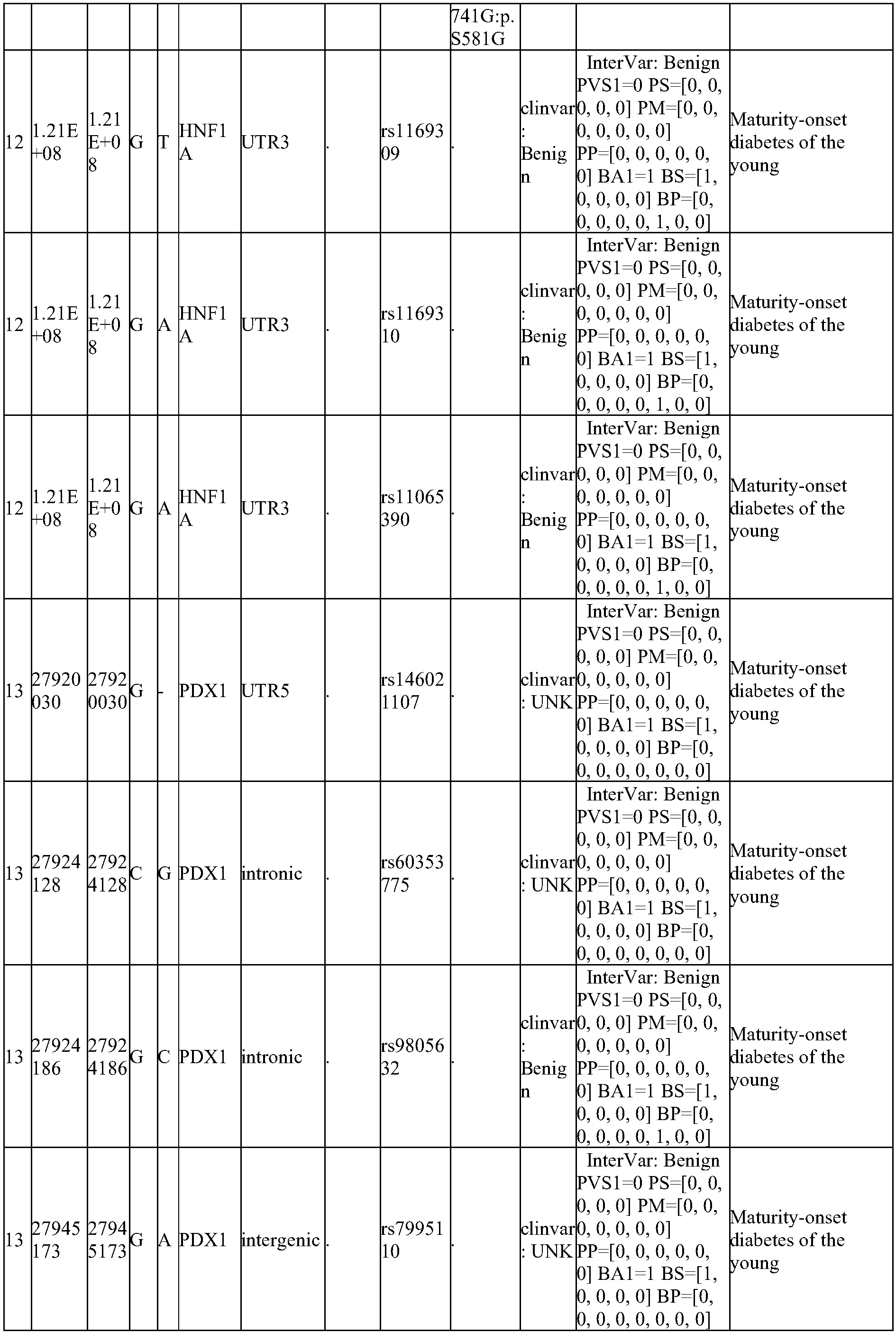

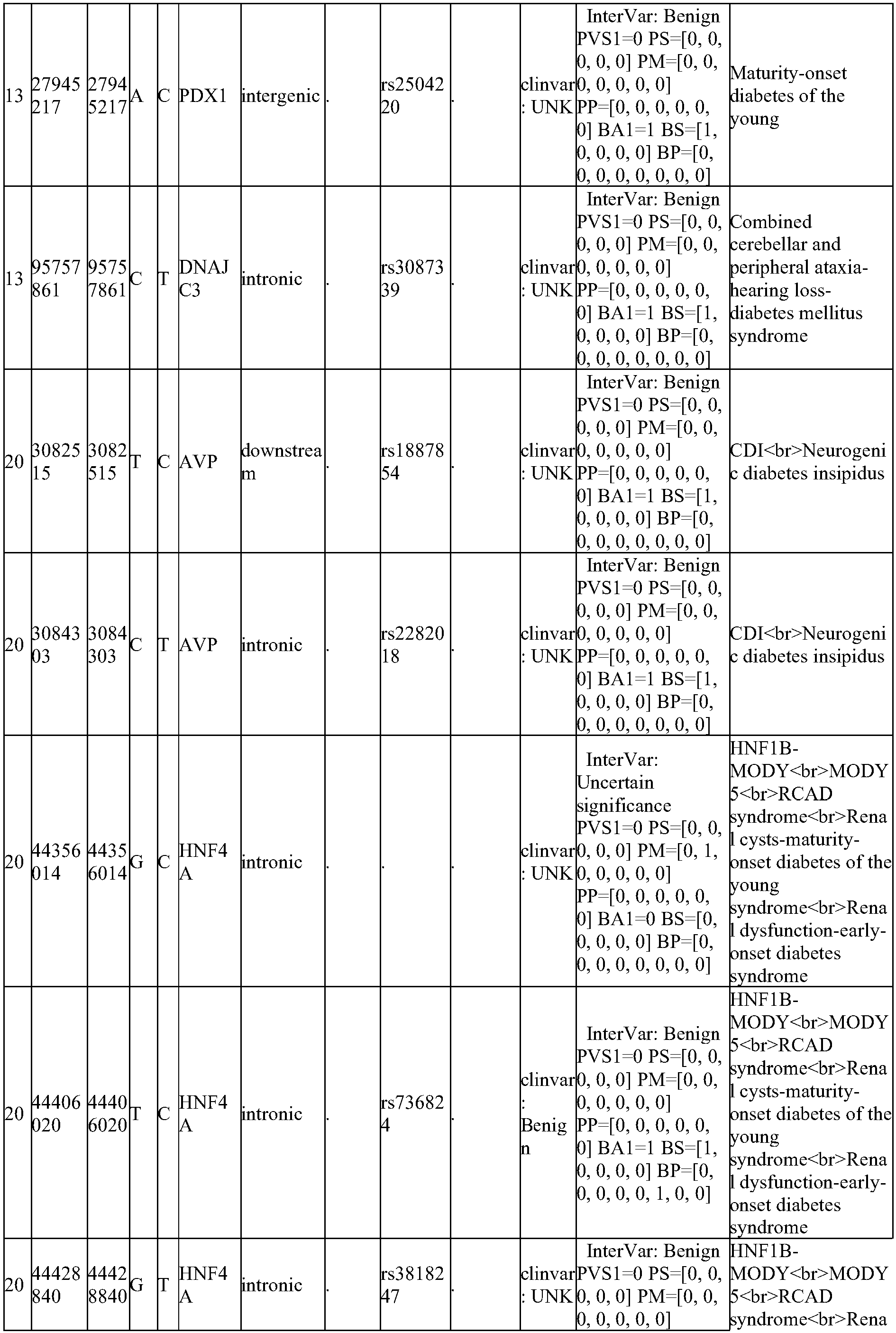

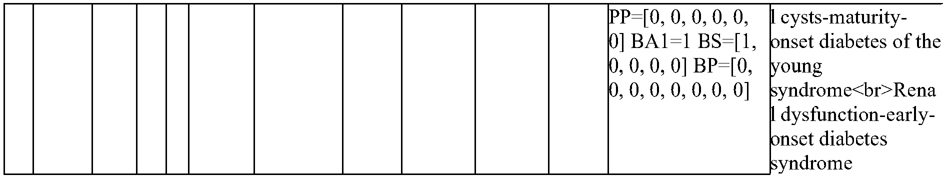

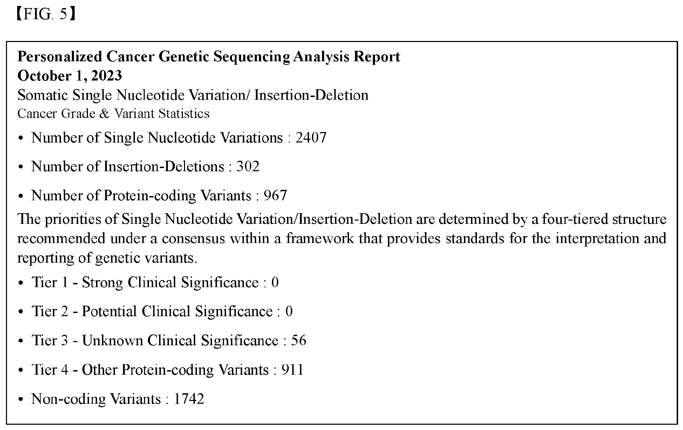

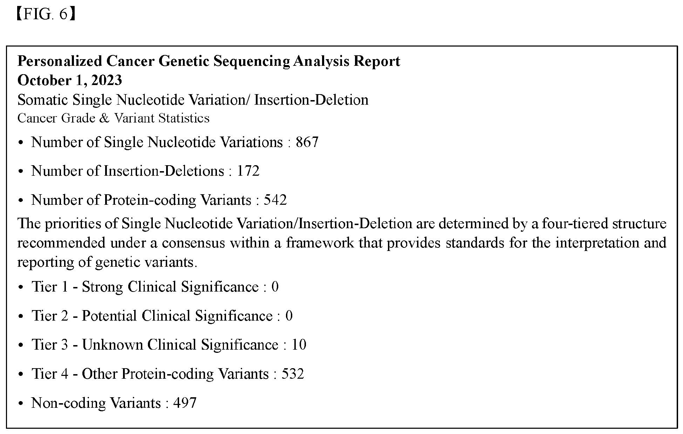

Additionally, when identifying markers related to pancreatic cancer, which is one of the most frequently occurring carcinomas in diabetes patients, the mutation status of pancreatic cancer-related genes indicated in gray could be confirmed as shown in Table 4; although there were no mutations corresponding to Tier 1 or Tier 2, which are mutation criteria related to disease diagnosis and treatment decisions, numerous cases corresponding to Tier 3 mutations with unknown clinical significance that are of prophylactic interest for the future were identified (FIG. 5), and it was confirmed that these occurred more than 10 times more frequently compared to the borderline diabetes sample group in FIG. 6 and Table 5.

**[Table 4]**

| | **SYMB OL** | **PROTEIN_CHAN GE** | **GENE_NAME** | **PROTEIN_D OMAIN** | **CONSEQUE NCE** |
|---|---|---|---|---|---|
| **1** | SPRED 1 | p.Ser37Gly | sprouty related EVH1 domain containing 1 | WH1 domain | missense_vari ant |
| **2** | SPRED 1 | p.Ser41Cys | sprouty related EVH1 domain containing 1 | WH1 domain | missense_vari ant |
| **3** | PALB2 | p.Tyr743Cys | partner and localizer of BRCA2 | | missense_vari ant |
| **5** | STAT3 | p.Thr663Ser | signal transducer and activator of transcription 3 | SH2 domain | missense_vari ant |
| **6** | CYLD | p.Asp32Glu | CYLD lysine 63 deubiquitinase | | missense_vari ant |
| **7** | CYLD | p.Leu567_Ser568del insPheCys | CYLD lysine 63 deubiquitinase | | missense_vari ant |
| 8 | FGFR2 | p.Ser354Thr | fibroblast growth factor receptor 2 | Immunoglobuli n I-set domain | missense_vari ant |
| **1 0** | NOTC H1 | p.His1735Gln | notch receptor 1 | | missense_vari ant |
| **1 1** | NOTC H1 | p.Thr1351Ala | notch receptor 1 | | missense_vari ant |
| **1 2** | KMT2C | p.Gly838Ser | lysine methyltransferase 2C | | missense_vari ant |
| **1 3** | KMT2 D | p.Gln3839ArgfsTer4 1 | lysine methyltransferase 2D | | frameshift_va riant |
| **1 4** | CUX1 | p.Ser637Asn | cut like homeobox 1 | | missense_vari ant |
| **1 5** | SMAD3 | p.Leu153Met | SMAD family member 3 | | missense_vari ant |
| **1 6** | ATRX | p.Glu929Gln | ATRX chromatin remodeler | | missense_vari ant |
| **1 7** | ARHG AP26 | p.Val699Ile | Rho GTPase activating protein 26 | | missense_vari ant |
| **1 8** | KDM6 A | p.Val156Ala | lysine demethylase 6A | Tetratricopepti de repeat | missense_vari ant |
| **1 9** | KDM6 A | p.Arg664Gln | lysine demethylase 6A | | missense_vari ant |
| **2 0** | KDM6 A | p.Thr668Ser | lysine demethylase 6A | | missense_vari ant |
| **2 1** | TRIM3 3 | p.Asp1050Glu | tripartite motif containing 33 | | missense_vari ant |
| **2 2** | NDRG1 | p.Gln127Lys | N-myc downstream regulated 1 | Ndr family | missense_vari ant |
| **2 3** | NDRG1 | p.Val125Ile | N-myc downstream regulated 1 | Ndr family | missense_vari ant |
| **2 4** | QKI | p.Ala225del | QKI, KH domain containing RNA binding | | inframe_delet ion |
| **2 5** | DLC1 | p.Val1486Ile | DLC1 Rho GTPase activating protein | START domain | missense_vari ant |
| **2 6** | KAT6B | p.Met363Val | lysine acetyltransferase 6B | | missense_vari ant |
| **2 7** | KDM5 C | p.Val1492Ala | lysine demethylase 5C | | missense_vari ant |
| **2 8** | PATZ1 | p.Leu533Pro | POZ/BTB and AT hook containing zinc finger 1 | | missense_vari ant |
| 2 **9** | PATZ1 | p.Pro531Ser | POZ/BTB and AT hook containing zinc finger 1 | | missense_vari ant |
| **3 0** | DMBT1 | p.Arg670Cys | deleted in malignant brain tumors 1 | Scavenger receptor cysteine-rich domain | missense_vari ant |
| **3 1** | S100A4 | p.Asn68Ser | S100 calcium binding protein A4 | | missense_vari ant |
| **3 2** | TGFBR 3 | p.Gly765Arg | transforming growth factor beta receptor 3 | | missense_vari ant |
| **3 3** | CIR1 | p.Arg183Gln | corepressor interacting with RBPJ, CIR1 | | missense_vari ant |
| **3 4** | RBMS3 | p.Pro437Gln | RNA binding motif single stranded interacting protein 3 | | missense_vari ant, splice_region variant |
| **3 5** | SHISA3 | p.Phe235Cys | shisa family member 3 | | missense_vari ant |
| **3 6** | TGFBI | p.Arg257Trp | transforming growth factor beta induced | Fasciclin domain | missense_vari ant, splice_region variant |
| **3 7** | AZGP1 | p.Pro20Leu | alpha-2-glycoprotein 1, zinc-binding | | missense_vari ant |
| **3 8** | CELF2 | | CUGBP Elav-like family member 2 | | frameshift_va riant, splice_region variant |
| **3 9** | VWA5 A | p.Ile582Thr | von Willebrand factor A domain containing 5A | | missense_vari ant |
| **4 0** | SUSD2 | p.Arg34His | sushi domain containing 2 | Somatomedin B domain | missense_vari ant |
| **4 1** | SUSD2 | p.Gly70Val | sushi domain containing 2 | | missense_vari ant |
| **4 2** | CUL4A | p.Phe394Tyr | cullin 4A | Cullin family | missense_vari ant |
| **4 3** | LYN | p.Phe165LeufsTer42 | LYN proto-oncogene, Src family tyrosine kinase | SH2 domain | frameshift_va riant |
| **4 4** | ROS1 | p.Cys1117Tyr | ROS proto-oncogene 1, receptor tyrosine kinase | | missense_vari ant |
| **4 5** | ERBB3 | p.Arg1202Trp | erb-b2 receptor tyrosine kinase 3 | | missense_vari ant |
| **4 6** | NSD2 | p.Pro234His | nuclear receptor binding SET domain protein 2 | PWWP domain | missense_vari ant |
| **4 7** | TLX1 | p.Leu276Met | T cell leukemia homeobox 1 | | missense_vari ant |
| **4 8** | ETV4 | p.Val448Ile | ETS variant transcription factor 4 | | missense_vari ant |
| **4 9** | NFATC 2 | p.Pro237Ser | nuclear factor of activated T cells 2 | | missense_vari ant |
| **5 0** | HGF | p.Gln173Arg | hepatocyte growth factor | Kringle domain | missense_vari ant |
| **5 1** | PBK | p.Ser57Phe | PDZ binding kinase | Protein kinase domain | missense_vari ant |
| **5 2** | RAB1A | p.Gly201Ser | RAB1A, member RAS oncogene family | | missense_vari ant |
| **5 3** | NNT | p.Leu822PhefsTer8 | nicotinamide nucleotide transhydrogenase | NAD(P) transhydrogena se beta subunit | frameshift_va riant |
| **5 4** | NNT | p.Thr823Ile | nicotinamide nucleotide transhydrogenase | NAD(P) transhydrogena se beta subunit | missense_vari ant |
| **5 5** | RUNX2 | p.Thr9Ser | RUNX family transcription factor 2 | | missense_vari ant |
| **5 6** | BRF2 | p.Pro351Leu | BRF2 RNA polymerase III transcription initiation factor subunit | | missense_vari ant |

**[Table 5]**

| | **SYMB OL** | **PROTEIN_CHA NGE** | **GENE_NAM E** | **PROTEIN_DO MAIN** | **CONSEQUEN CE** |
|---|---|---|---|---|---|
| 1 | SMAD3 | p.Leu153Met | SMAD family member 3 | | missense_variant |
| 2 | ATRX | p.Glu929Gln | ATRX chromatin remodeler | | missense_variant |
| 3 | PAX6 | p.Ter437GluextTe r14 | paired box 6 | | stop_lost |
| 4 | DMBT1 | p.Arg670Cys | deleted in malignant brain tumors 1 | Scavenger receptor cysteine-rich domain | missense_variant |
| 5 | DMBT1 | p.Pro794Leu | deleted in malignant brain tumors 1 | Scavenger receptor cysteine-rich domain | missense_variant |
| 6 | PTPRG | p.Gly1146Arg | protein tyrosine phosphatase receptor type G | | missense_variant |
| 7 | SHISA3 | p.Phe235Cys | shisa family member 3 | | missense_variant |
| 8 | TGFBI | p.Arg257Trp | transforming growth factor beta induced | Fasciclin domain | missense_variant , splice_region_va riant |
| 9 | SUSD2 | p.Arg34His | sushi domain containing 2 | Somatomedin B domain | missense_variant |
| 1 0 | GOLM1 | p.Pro265His | golgi membrane protein 1 | | missense_variant |

Based on the above results, the Tier 3 stage is a part that can be observed as disease recurrence and long-term outcomes of treatment in relation to health sustainability; therefore, it can be said that these analysis results can positively contribute to clients and patients by allowing them to predict the changing status of the disease through periodic monitoring of said part and to prepare preventive and management measures accordingly.

Furthermore, specifically, by enabling the observation of types of genetic mutations indicating the association between obesity and diabetes and the potential as future risk factors for pancreatic cancer, results confirming the applicability to diagnosis related to prognosis or prediction were derived; and, similar result patterns of genetic mutations inherited through the maternal line were confirmed, demonstrating the possibility of early diagnosis and preventive medical application for diseases that can genetically occur between family members in relation to chronic diseases such as diabetes.

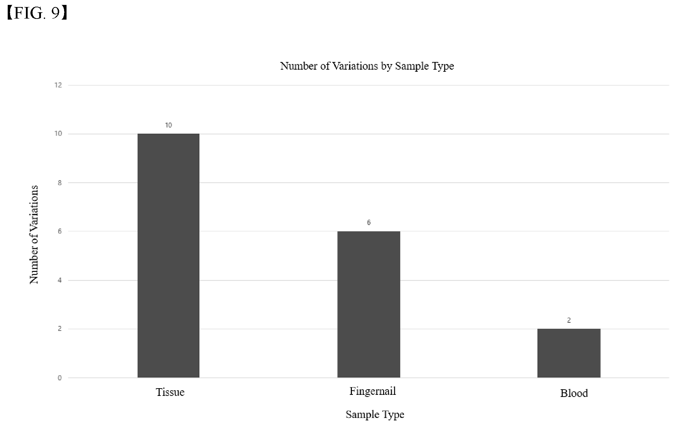

As a result of analyzing somatic variants in three types of specimens nail, blood, and surgical tissue from bladder cancer patients, more bladder cancer-related somatic mutations were discovered in nail samples compared to whole blood. As shown in Table 6 and FIG. 9 below, when comparing each mutation, it was found that the nail samples shared more common mutations with the tissue samples.

**[Table 6]**

| Variant info | | | | Sample type | | |
|---|---|---|---|---|---|---|
| Gene | Ref | Alt | CDS change | Tissue | Nail | Bloo d |
| MSH6 | CT | C | c.4002-10del | o | o | o |
| MSH6 | AT | A | c.3557-4del | o | o | |
| PIK3CA | CT | C | c.562+88del | o | o | o |
| TP53 | G | A | c.574C>T | o | | |
| CDKN2 A | G | T | c.69C>A | o | | |
| ERBB2 | G | A | c.1738G>A | o | | |
| EGFR | C | T | c.241-26C>T | o | | |
| PMS2 | GAA | G | c.706-5_706-4del | o | | |
| ERBB2 | TA | T | | o | | |
| ERBB2 | G | A | c.2209-93G>A | o | | |
| KRAS | T | G | c.*3901A>C | | o | |
| EGFR | G | A | c.402G>A | | o | |
| MLH1 | AAC | A | c.1409+1226_1409+1227del | | o | |

Additionally, in the case of individual specific mutations detected by WES, the depth of coverage is relatively lower compared to targeted sequencing, and because the number of analyzed genes is large, variants of unknown significance (VOUS) may be detected; therefore, if a specific genetic disease is suspected, target genes and mutations can be secondary-screened through nail specimen-based targeted sequencing (Panels) targeting specific diseases, and then a panel kit can be manufactured to obtain high-depth data of 1000X or more..

In the report generation stage, the results delivered from the nail sample analysis operation and the results delivered from the clinical information analysis operation may be aggregated and provided in the form of an HTML5-based responsive web, which can be provided to terminals accessible by individuals in the case of frontline screening centers or local hospital DTC services. Each of these components may refer to software or hardware such as an FPGA (Field Programmable Gate Array) or an ASIC (Application-Specific Integrated Circuit), but is not limited to software or hardware; they may be configured to reside in an addressable storage medium or may be configured to execute one or more processors consisting of subdivided components or a plurality of components.

In addition, the data collected by the above method can be advanced through machine learning for big data, and supervised learning or unsupervised learning can be utilized depending on conditions; a process to increase reliability can be performed through supervised learning that optimizes variables related to diseases by adding clinical information (blood pressure, blood glucose, smoking, drinking, disease history, etc.), mutation information of chronic diseases or cancer genes, psychological questionnaire information, and gut microbiome information, under a state where specific answers exist. As more individual samples and clinical information are accumulated, they can be utilized as deep learning algorithms to increase accuracy and to perform correlation analysis between clinical findings and the discovery of mutant genes with accurate prognostic predictions for monitoring.

Based on such an analysis system, it is possible to provide customized checkup information or care methods according to diagnosis for each requester every month, based on uniquely stored individual information. For example, an online result report can be provided to the institution, checkup center, hospital, etc., where the requester submitted a nail sample, and the institution, checkup center, hospital, etc., provided with the online result report can grasp the requester's situation and recommend an intensive diagnosis if it is predicted that an additional examination is necessary, and can provide the requester with superior wellness-related counseling services, genetic health checkup counseling services, or medical services.

## Claims

1. A nail-based NGS (Next Generation Sequencing) analysis method for predicting or diagnosing a disease, comprising the steps of:
(a) isolating gDNA from a nail; and
(b) analyzing the gDNA isolated in step (a) by NGS.

2. The nail-based NGS analysis method according to claim 1, wherein the nail is at least one selected from the group consisting of a fingernail and a toenail.

3. The nail-based NGS analysis method according to claim 1, wherein the NGS in step (a) involves isolating the gDNA from the nail by a forensic method.

4. The nail-based NGS analysis method according to claim 1, wherein the NGS analysis in step (b) is performed by an Illumina sequencing method.

5. The nail-based NGS analysis method according to claim 1, further comprising the step of (c) mapping reads of a region corresponding to a gene in the gDNA isolated from the nail.

6. The nail-based NGS analysis method according to claim 5, wherein the mapping in step (c) comprises sequencing the gDNA isolated from the nail and then aligning and mapping each read based on a reference genome.

7. The nail-based NGS analysis method according to claim 5, further comprising the step of (d) calculating the expressivity of a gene associated with the onset of a disease.

8. The nail-based NGS analysis method according to claim 7, wherein the calculating in step (d) comprises calculating the expressivity of the gene associated with the onset of the disease by identifying probabilities that data to be analyzed corresponds to each of genotypes regarding a gene to be analyzed.

9. The nail-based NGS analysis method according to claim 7, wherein the calculating in step (d) comprises identifying positions and sequences with mutations, or detecting and identifying somatic mutations or germline mutations.

10. The nail-based NGS analysis method according to claim 7, wherein the calculating in step (d) is performed by a variant caller algorithm.

11. The nail-based NGS analysis method according to claim 7, further comprising the step of (e) constructing a genomic database.

12. The nail-based NGS analysis method according to claim 11, wherein constructing the genomic database in step (e) comprises constructing a genomic information catalog of an individual for the prevention or prediction of a disease through identification of a germline mutation and a somatic mutation.

13. A system for predicting or diagnosing a disease using an NGS analysis method, comprising the steps of:
(a1) loading a computer program to be executed by one or more processors;
(b1) providing a memory for storing data in which genotypes regarding a genome to be analyzed are determined;
(c1) generating an individual genetic analysis result report based on the stored data; and
(d1) a system construction unit for constructing a genomic database of the subject to be analyzed, and a report generation step for providing information from the system construction unit to the subject.

14. The system for predicting or diagnosing a disease according to claim 13, wherein the one or more processors in step (a1) are configured to perform operations comprising:
(i) an operation step of obtaining and storing data by receiving a nail sample, general information, or clinical information of a subject,
(ii) a gDNA sample analysis operation step of aligning and mapping DNA fragments obtained from the nail sample through NGS to a reference genome sequence representing a species, and
(iii) a clinical information analysis operation step of calculating the expressivity of genotypes from the data of the subject.

15. The system for predicting or diagnosing a disease according to claim 13, wherein the nail is at least one selected from the group consisting of a fingernail and a toenail.

16. The system for predicting or diagnosing a disease according to claim 14, wherein the general information includes age, gender, family history, or lifestyle.

17. The system for predicting or diagnosing a disease according to claim 14, wherein the clinical information includes blood pressure, blood sugar, smoking, drinking, disease history, vaccine and immunization information, regular checkup items, types and dosages of prescribed drugs, side effect history, or allergic reactions.

18. The system for predicting or diagnosing a disease according to claim 13, wherein the genetic analysis result report includes an individual's constitution, customized nutrition and diet, exercise, stress relief and psychological mental health management, recommended activities, combinations of health functional foods, supplements, regular health checkup cycles and methods, or traceable health goals and progress.

19. The nail-based NGS analysis method according to claim 1, wherein the disease is at least one selected from the group consisting of aging, obesity, hypertension, diabetes, hyperlipidemia, cancer, sarcopenia, osteoporosis, lung disease, cardiovascular disease, brain disease, liver disease, dementia, and Alzheimer's disease.

20. The system for predicting or diagnosing a disease according to claim 13, wherein the disease is at least one selected from the group consisting of aging, obesity, hypertension, diabetes, hyperlipidemia, cancer, sarcopenia, osteoporosis, lung disease, cardiovascular disease, cerebrovascular disease, liver disease, dementia, and Alzheimer's disease.
